(19) Europäisches Patentamt
European Patent Office
Office européen des brevets

(11) EP 0 816 329 A1

(12) EUROPÄISCHE PATENTANMELDUNG

(43) Veröffentlichungstag:
**07.01.1998 Patentblatt 1998/02**

(21) Anmeldenummer: **97108869.5**

(22) Anmeldetag: **03.06.1997**

(51) Int. Cl.[6]: **C07C 233/55**, C07C 235/38, A61K 31/165, A61K 31/19, C07D 333/40, C07C 275/42, C07C 233/63, C07D 307/68, C07D 333/28, C07C 233/81, C07C 233/54, C07C 235/24, C07C 235/56, C07C 255/57

(84) Benannte Vertragsstaaten:
**AT BE CH DE DK ES FI FR GB GR IE IT LI LU NL PT SE**

(30) Priorität: **18.06.1996 DE 19624155**

(71) Anmelder:
**HOECHST AKTIENGESELLSCHAFT**
**65929 Frankfurt am Main (DE)**

(72) Erfinder:
- **Hemmerle, Horst, Dr.**
  **64653 Lorsch (DE)**
- **Henke, Stephan, Dr.**
  **65719 Hofheim (DE)**
- **Below, Peter, Dr.**
  **60529 Frankfurt (DE)**
- **Burger, Hans-Jörg, Dr.**
  **65719 Hofheim (DE)**
- **Herling, Andreas, Dr.**
  **65520 Bad Camberg (DE)**
- **Jähne, Gerhard, Dr.**
  **65929 Frankfurt (DE)**

# (54) Substituierte Benzoesäurederivate, Verfahren zu ihrer Herstellung und die Anwendung der Verbindungen zur Behandlung von Krankheiten

(57) Es werden substituierte Benzoesäurederivate der Formel I,

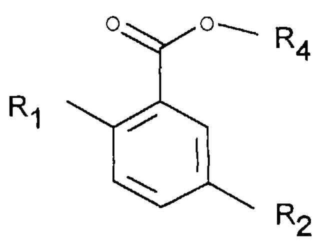

I

worin die Reste die angegebenen Bedeutungen haben und ein Verfahren zu deren Herstellung beschrieben. Die Verbindungen eignen sich zur Behandlung von Typ II Diabetes.

EP 0 816 329 A1

## Beschreibung

Das Krankheitsbild des Diabetes ist durch erhöhte Blutzuckerwerte gekennzeichnet. Beim insulinpflichtigen oder Typ I Diabetes ist die Ursache das Absterben der insulinproduzierenden β-Zellen des Pankreas; die Behandlung erfolgt daher durch Insulingabe ( Substitutionstherapie ). Der nicht-insulinabhängige oder Typ II Diabetes ist dagegen durch eine verminderte Insulinwirkung auf Muskel- und Fettgewebe ( Insulinresistenz ) und eine gesteigerte Glucoseproduktion der Leber gekennzeichnet. Die Ursachen dieser Stoffwechselstörungen sind noch weitgehend ungeklärt. Die etablierte Therapie mit Sulfonylharnstoffen versucht die Insulinresistenz durch Steigerung der körpereigenen Insulinfreisetzung zu kompensieren, führt jedoch nicht in allen Fällen zu einer Normalisierung des Blutzuckerspiegels und vermag das Fortschreiten der Krankheit nicht aufzuhalten; viele Typ II Diabetiker werden schließlich durch "Erschöpfung" der β-Zellen insulinpflichtig und leiden an Spätschäden wie Katarakten, Nephropathien und Angiopathien. Neue Therapieprinzipien zur Behandlung des Typ II Diabetes sind deshalb wünschenswert.

Die Konzentration der Blutglucose im nüchternen Zustand wird durch die Glucoseproduktion der Leber bestimmt. Verschiedene Arbeitsgruppen konnten zeigen, daß die Erhöhung der Blutzuckerwerte bei Typ II Diabetes mit einer proportional erhöhten Glucoseabgabe aus der Leber korrelieren. Die von der Leber in das Blut abgegebene Glucose kann sowohl durch Abbau von Leber-Glycogen (Glycogenolyse) als auch durch Gluconeogenese gebildet werden. Glucose-6-Phosphat ist das gemeinsame Endprodukt sowohl der Gluconeogenese als auch der Glycogenolyse. Der terminale Schritt der hepatischen Freisetzung von Glucose aus Glucose-6-Phosphat wird von der Glucose-6-Phosphatase (EC 3.1.3.9) katalysiert. Die Glucose-6-Phosphatase stellt einen im endoplasmatischen Reticulum (ER) vorkommenden Multienzym-Komplex dar. Dieser Enzym-Komplex besteht aus einer in der ER-Membran vorliegenden Glucose-6-Phosphat-Translocase, einer auf der luminalen Seite des endoplasmatischen Reticulum lokalisierten Glucose-6-Phoshatase und einer Phosphat-Translocase [für eine Übersicht siehe: Ashmore J. und Weber G., "The Role of Hepatic Glucose-6-phosphatase in the Regulation of Carbohydrate Metabolism", in Vitamins und Hormones, Vol XVII (Harris R.S., Marrian G.F., Thimann K.V., Edts.), 92-132, (1959); Burchell A., Waddell I.D., "The molecular basis of the hepatic microsomal glucose-6-phosphatase system", Biochim. Biophys. Acta 1092, 129-137, (1990)]. Die vorliegende umfangreiche Literatur zeigt, daß unter allen untersuchten Bedingungen, die im Tierversuch zu erhöhten Blutzuckerwerten führen, z. B. Streptozotocin, Alloxan, Cortison, Thyroid-Hormone und Hungern, die Aktivität dieses Multienzym-Komplexes ebenfalls erhöht ist. Darüber hinaus weisen zahlreiche Untersuchungen darauf hin, daß die bei Typ II Diabetikern beobachtete erhöhte Glucose-Produktion mit einer erhöhten Glucose-6-Phosphatase-Aktivität verbunden ist.

Die Bedeutung des Glucose-6-Phosphatase-Systems für eine normale Glucose-Homeostase wird ferner unterstrichen durch die hypoglykämischen Symptome von Patienten mit Glykogen-Speicherkrankheit Typ Ib, denen die Translocase-Komponente des Glucose-6-Phosphat-Systems fehlt.

Eine Verringerung der Glucose-6-Phosphatase-Aktivität durch geeignete Wirkstoffe (Inhibitoren) sollte zu einer verringerten hepatischen Glucose-Freisetzung führen. Diese Wirkstoffe sollten in der Lage sein, die Glucose-Produktion der Leber dem effektiven peripheren Verbrauch anzupassen. Die dadurch im nüchternen Zustand von Typ II Diabetikern gesenkten Blutglucose-Werte dürften darüber hinaus auch eine präventive Wirkung im Hinblick auf diabetische Spätschäden haben. In der Literatur ist eine Reihe von unspezifischen Inhibitoren der Glucose-6-Phosphatase beschrieben worden wie z.B. Phlorrhizin [Soodsma,J.F., Legler,B. und Nordlie, R.C., J.Biol. Chem. 242, 1955-1960, (1967)], 5,5`-Dithio-bis-2-nitro-benzoesäure [Wallin, B.K. und Arion, W.J., Biochem. Biophys. Res. Commun. 48, 694-699, (1972)], 2,2`-Di-isothiocyanato-stilben und 2-Isothiocyanato-2`-acetoxy-stilben [Zoccoli, M.A. und Karnowski, M.L., J. Biol. Chem. 255, 1113-1119, (1980)]. Die ersten therapeutisch verwertbaren Inhibitoren des Glucose-6-Phosphatase-Systems sind in EP 587 087 und EP 587 088 beschrieben.

Es wurde nun gefunden, daß bestimmte 2,5-substituierte Benzoesäuren bzw. deren Ester, wie z. B. Verbindung 43, sehr gute Hemmer des Glucose-6-Phosphatase-Systems sind und im Vergleich zu bisherigen bekannten Inhibitoren diese Enzymsystems eine sehr gute Bioverfügbarkeit aufweisen.

Die Synthese von an der 5-Position mit Phenyl bzw. substituierten Phenyl und der 2-Position mit NH-Acetyl- bzw. NH-Benzoyl-substituierter Benzoesäurederivate wird in E. Ota et al., Yuki Gosei Kyokai Shi, 28 (3), 341-345 (1970) und G. I. Migachev et al., Zh, Vses. Khim. O-Va, 24(1), 93-94 (1979) beschrieben.

Die Erfindung betrifft daher Verbindungen der Formel I

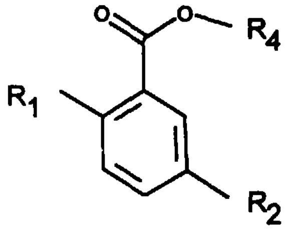

I

worin die Reste die folgende Bedeutung haben:

$R^1$

1. $C_1$-$C_{10}$-Alkyl,
2. $C_1$-$C_{10}$-Perfluoralkyl,
3. $C_1$-$C_{10}$-Hydroxyalkyl,
4. $C_2$-$C_{10}$-Alkenyl,
5. $C_2$-$C_{10}$-Alkinyl,
6. $C_1$-$C_{10}$-Alkyl-$C_6$-$C_{12}$-Aryl, wobei eine oder mehrere Methyleneinheiten durch O oder S ersetzt sein können,
7. $C_2$-$C_{10}$-Alkinyl-$C_6$-$C_{12}$-Aryl,
8. $C_6$-$C_{12}$-Aryl,
9. $C_1$-$C_9$-Heteroaryl,
10. $C_1$-$C_{10}$-Alkoxy, wobei der Alkylrest geradkettig oder verzweigt oder ab $C_3$-Alkoxy auch cyclisch sein kann,
11. O-$C_1$-$C_{10}$-Alkyl-$C_6$-$C_{12}$-Aryl, beispielsweise Benzyloxy,
12. einen Rest 1., 4., 5., 6., 7., 8., 9. substituiert mit einem oder mehreren Resten aus der Reihe CN, Nitro, Trifluormethyl, Halogen, Hydroxy-, Phenyl, $NH_2$, NH($C_1$-$C_{10}$-alkyl), N($C_1$-$C_{10}$-alkyl)$_2$, $C_1$-$C_{10}$-Alkoxy- (wobei der Alkylrest geradkettig oder verzweigt, oder ab $C_3$-Alkoxy auch cyclisch sein kann);

$R^2$

1. $C_1$-$C_{10}$-Alkyl,
2. $C_2$-$C_{10}$-Alkenyl,
3. $C_2$-$C_{10}$-Alkinyl,
4. $C_1$-$C_{10}$-Alkyl-$C_6$-$C_{12}$-Aryl,
5. $C_2$-$C_{10}$-Alkinyl-$C_6$-$C_{12}$-Aryl,
6. $C_6$-$C_{12}$-Aryl,
7. $C_2$-$C_{10}$-Alkinyl-$C_3$-$C_8$-Cycloalkenyl,
8. $C_1$-$C_9$-Heteroaryl;
9. NH-C0-$R^3$,
10. $NHSO_2$-$C_6$-$C_{12}$-Aryl, wobei der Aryl-Teil gegebenenfalls mit 1, 2 oder 3 Resten aus der Reihe $NO_2$, Halogen, Trifluormethyl, CN, NH($C_1$-$C_{10}$-Alkyl), N($C_1$-$C_{10}$-Alkyl)$_2$, $C_1$-$C_{10}$-Alkoxy, Hydroxy substituiert ist,
11. $C_1$-$C_{10}$-Alkanoyl;
12. einen Rest 1. - 8. ein- oder mehrfach substituiert mit $NH_2$, NH($C_1$-$C_{10}$-alkyl), N($C_1$-$C_{10}$-alkyl)$_2$, Carboxy, Halogen, Hydroxy, $C_1$-$C_6$-Alkoxy;

$R^3$

1. $C_1$-$C_{10}$-Alkyl,
2. $C_1$-$C_{10}$-Alkyl-$C_6$-$C_{12}$-Aryl,
3. $C_1$-$C_{10}$-Alkyl-($C_6$-$C_{12}$-Aryl)$_2$,
4. $C_1$-$C_{10}$-Alkyl-$C_1$-$C_9$-Heteroaryl,
5. $C_1$-$C_{10}$-Alkyl-($C_1$-$C_9$-Heteroaryl)$_2$,
6. NH-$C_6$-$C_{12}$-Aryl,

7. NH-$C_1$-$C_9$-Heteroaryl,
9. $C_2$-$C_{10}$-Alkenyl-$C_6$-$C_{12}$-Aryl,
10. $C_6$-$C_{12}$-Aryl,
11. $C_3$-$C_8$-Cycloalkyl,
12. $C_3$-$C_8$-Cycloalkyl-$C_6$-$C_{12}$-aryl,
13. $C_1$-$C_9$-Heteroaryl,
14. $C_1$-$C_{10}$-Alkyl-$C_3$-$C_8$-cycloalkyl,
15. Adamantyl,
16. C-O-$C_1$-$C_{10}$-Alkyl-$C_6$-$C_{12}$-Aryl, z.B. C-O-Benzyl,
17. C-O-$C_6$-$C_{12}$-Aryl, beispielsweise C-O-Phenyl,
18. Fluorenonyl

wobei die $C_6$-$C_{12}$-Aryl- und $C_1$-$C_9$-Heteroarylreste ein- oder mehrfach mit einem Rest aus der Reihe Hydroxy-, Halogen, $C_1$-$C_4$-Alkoxy, $NH_2$, NH($C_1$-$C_{10}$-alkyl), N($C_1$-$C_{10}$-alkyl)$_2$ $C_1$-$C_{10}$-Chloralkyl, $NO_2$, Carboxy, Carbamido, CN substituiert sein können;

$R^4$

1. Wasserstoff,
2. $C_1$-$C_{10}$-Alkyl

sowie deren physiologisch verträglichen Salze,
ausgenommen die Verbindungen der Formel I in denen gleichzeitig bedeuten:

a)

$R^1$ Phenyl,
$R^2$ NH-Acetyl
$R^4$ Wasserstoff

b)

$R^1$ Phenyl, disubstiuiert mit Nitro und Carboxy
$R^2$ NH-Acetyl
$R^4$ Wasserstoff

c)

$R^1$ Phenyl,
$R^2$ NH-Benzoyl
$R^4$ Wasserstoff

Alkyl, Alkenyl und Alkinyl können geradkettig oder verzweigt sein. Entsprechendes gilt für davon abgeleitete Reste, wie Alkanoyl oder Alkoxy.

Unter Cycloalkyl werden auch alkylsubstituierte Ringe verstanden.

($C_6$-$C_{12}$)-Aryl ist beispielsweise Phenyl, Naphthyl oder Biphenylyl vorzugsweise Phenyl. Entsprechendes gilt für davon abgeleitete Reste, wie Aroyl oder Aralkyl.

Unter ($C_1$-$C_9$)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Weiter kann auch 1 oder beide Atome der Kondensationsstelle bicyclischer Reste (wie im Indolizinyl) ein N-Atom sein.

Dies sind z. B. Furanyl, Thienyl, Pyrrolyl, Imidazolyl, Pyrazolyl, Triazoly, Tetrazolyl, Oxazolyl, Isoxazolyl, Thiazolyl, Isothiazolyl Pyridyl, Pyrazinyl, Pyrimidinyl, Benzodioxolyl, Pyradiazinyl, Indolyl, Indazolyl, Chinolyl, Isochinolyl, Phthalazinyl, Chinoxalinyl, Chinazolinyl, Cinnolinyl, Dioxolanoyl, sowie davon abgeleitete Benzoannellierte Analoge.

Alkylaryl- bzw. Akylheteroarylreste können sowohl im Aryl/Heteroarylteil als auch im Alkylteil (z. B. mit OH) wie oben beschrieben substituiert sein.

Bevorzugt sind Verbindungen der Formel I , worin $R^1$ $C_1$-$C_{10}$-Alkyl, $C_6$-$C_{12}$-Aryl oder $C_1$-$C_9$-Heteroaryl bedeuten und gegebenenfalls wie oben definiert ein oder mehrfach substituiert sind, und deren physiologisch verträglichen

Salze.

Des weiteren sind Verbindungen der Formel I bevorzugt, worin $R^2$ $C_2$-$C_{10}$-Alkinyl, $C_2$-$C_{10}$-Alkinyl-$C_6$-$C_{12}$-Aryl, $C_6$-$C_{12}$-Aryl, die gegebenenfalls wie oben definiert ein oder mehrfach substiuiert sind , oder NH-C0-$R^3$ bedeutet und $R^3$ wie oben definiert ist sowie deren physiologisch verträglichen Salze.

Bevorzugt sind auch Verbindungen in denen $R^1$ $C_1$-$C_{10}$-Alkyl, $C_6$-$C_{12}$-Aryl oder $C_1$-$C_9$-Heteroaryl bedeuten und gegebenenfalls wie oben definiert ein oder mehrfach substituiert sind, $R^2$ $C_2$-$C_{10}$-Alkinyl, $C_2$-$C_{10}$-Alkinyl-$C_6$-$C_{12}$-Aryl, $C_6$-$C_{12}$-Aryl, die gegebenenfalls wie oben definiert ein oder mehrfach substiuiert sind , NH-C0-$R^3$, insbesondere NH-C0-$R^3$ bedeuten und $R^3$ und $R^4$ wie oben definiert sind sowie deren physiologisch verträglichen Salze.

Ganz besonders bevorzugt sind Verbindungen der Formel I in denen $R^2$ NH-CO-$R^3$ bedeutet und $R^3$ wie oben definiert ist sowie deren physiologisch verträglichen Salze.

Die erfindungsgemäßen Verbindungen der Formel I können, soweit sie eine Carboxylgruppe enthalten, Salze mit anorganischen oder organischen Basen bilden. Bevorzugt sind Salze mit anorganischen Basen, besonders die physiologisch unbedenklichen Alkalimetall-Salze, vor allem Natrium- und Kaliumsalze.

Die Verbindungen der Formel I hemmen das Glucose-6-Phosphatase System der Leber in Säugetieren. Die Verbindungen eigenen sich daher als Arzneimittel. Die Erfindung betrifft daher auch Arzneimittel auf Basis der Verbindungen der Formel, gegebenfalls in Form der physiologisch verträglichen Salze. Die Erfindung betrifft weiterhin die Anwendung von Verbindungen der Formel I oder der Salze zur Behandlung von Krankheiten, die mit einer erhöhten Aktivität des Glucose-6-Phosphatase-Systems verbunden sind.

Die Erfindung betrifft daher auch die Anwendung von Verbindungen der Formel I bzw. der Salze zur Behandlung von Krankheiten, die mit einer erhöhten Glucoseproduktion der Leber verbunden sind.

Die Erfindung betrifft außerdem die Anwendung von Verbindungen der Formel I bzw. der Salze zur Behandlung des Typs II Diabetes (nicht insulinabhängiger oder Altersdiabetes).

Die Erfindung beinhaltet weiterhin die Anwendung von Verbindungen der Formel I bzw. der Salze zur Herstellung von Arzneimitteln zur Behandlung des Diabetes und anderer Erkrankungen, die durch einen erhöhten Glucose-Ausstoß aus der Leber oder eine erhöhte Aktivität des Glucose-6-Phosphatase-Systems gekennzeichnet sind.

Die Wirkung der erfindungsgemäßen Verbindungen auf das Glucose-6-Phosphatase-System wurde in einem Enzymtest in Lebermikrosomen untersucht.

Für die Präparation der die Glucose-6-Phosphatase enthaltenden Mikrosomen-Fraktion wurden frische Leber-Organe von männlichen Wistar-Ratten verwendet und wie in der Literatur beschrieben aufgearbeitet [Canfield, W. K. Und Arion, W.J., J. Biol. Chem. 263, 7458-7460, (1988)]. Diese Mikrosomen-Fraktion kann bei -70°C mindestens 2 Monate lang ohne signifikamten Aktivitätsverlust aufbewahrt werden.

Bestimmung der biologischen Aktivität:

Der Nachweis der Glucose-6-Phosphatase-Aktivität wurde wie in der Literatur angegeben (Arion, W. J. in Methods Enzymol. 174, Academic Press 1989, Seite 58 - 67) durch Bestimmung des aus Glucose-6-Phosphat freigesetzten Phosphats durchgeführt. 0,1 ml Testansatz enthielten Glucose-6-Phosphat (1mMol/l), die Testsubstanz, 0,1 mg Mikrosomen-Fraktion und 100 mMol/l HEPES-Puffer (4-(2-Hydroxyethyl)piperazin-1-ethansulfonsäure), pH 7,0. Die Reaktion wurde durch Zugabe des Enzyms gestartet. Nach Ablauf von 20 Minuten bei Raumtemperatur wurde die Reaktion durch Zugabe von 0,2 ml Phosphat-Reagens gestoppt. Die Probe wurde 30 Minuten lang bei 37 °C inkubiert, und die Absorbtion (A) der blauen Farbe anschließend bei 570 nm gemessen. Die inhibitorische Wirksamkeit der Testsubstanz ergab sich durch Vergleich mit einer Kontroll-Reaktion, die keine Testsubstanz enthielt nach der Formel

$$\text{Prozent Hemmung} = \frac{\text{A(Kontrolle) - A(Testsubstanz)}}{\text{A(Kontrolle)}} \times 100$$

Die hemmende Wirkung der Testsubstanz (Verbindungen 1 - 43) wurde bei den in der nachfolgenden Tabelle 1 angegebenen Konzentrationen der Testsubstanz ermittelt.

Tabelle 1:

| Nr. | Struktur | Summenformel | MS (M+H+) | % Hemmung | Inhibitorkonzentration [µM] | Herstellungsverfahren |
|---|---|---|---|---|---|---|
| 1 | 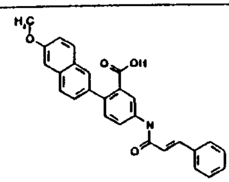 | C27H21NO4 | 424 | 53 | 100 | 2 |
| 2 | 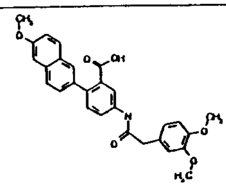 | C28H25NO6 | 472 | 60 | 100 | 2 |
| 3 | 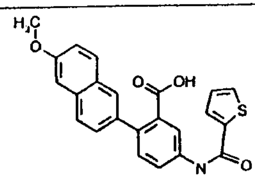 | C23H17NO4S | 404 | 93 | 100 | 2 |
| 4 | 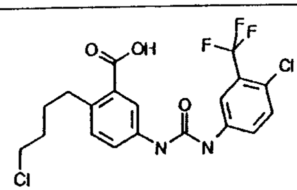 | C19H17Cl2F3N2O3 | 449 | 66 | 100 | 4 |
| 5 | 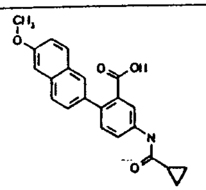 | C22H19NO4 | 362 | 51 | 100 | 2 |

| | | | | | | |
|---|---|---|---|---|---|---|
| 6 | 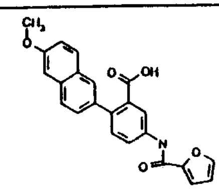 | C23H17NO5 | 388 | 61 | 100 | 2 |
| 7 | 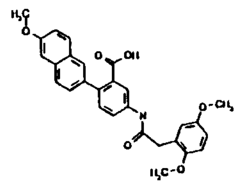 | C28H25NO6 | 472 | 63 | 100 | 2 |
| 8 | 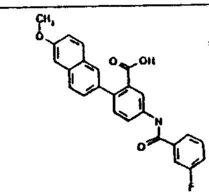 | C25H18FNO4 | 416 | 77 | 100 | 2 |
| 9 | 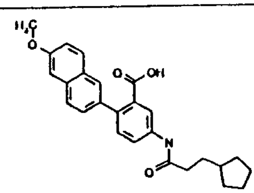 | C26H27NO4 | 418 | 64 | 100 | 2 |
| 10 | 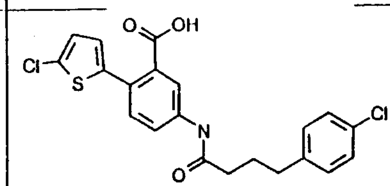 | C21H17Cl2NO3S | 434 | 62 | 100 | 2 |
| 11 | 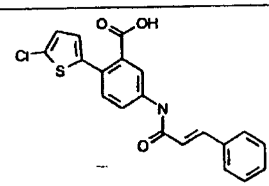 | C20H14ClNO3S | 384 | 57 | 100 | 2 |

| | | | | | | |
|---|---|---|---|---|---|---|
| 12 | 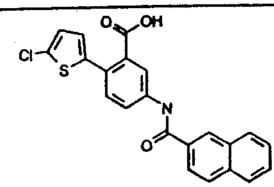 | C22H14ClNO3S | 408 | 71 | 100 | 2 |
| 13 | 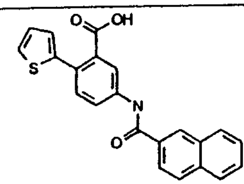 | C22H15NO3S | 374 | 53 | 100 | 2 |
| 14 | 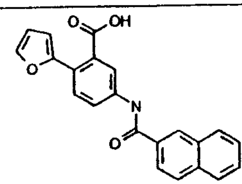 | C22H15NO4 | 358 | 57 | 100 | 2 |
| 15 | 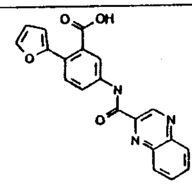 | C20H13N3O4 | 360 | 63 | 100 | 2 |
| 16 | 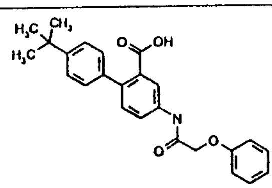 | C25H25NO4 | 404 | 50 | 100 | 2 |
| 17 | 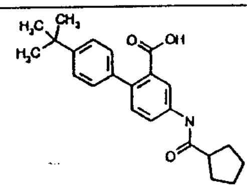 | C23H27NO3 | 366 | 53 | 100 | 2 |

| | | | | | | |
|---|---|---|---|---|---|---|
| 18 | 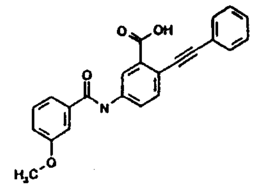 | C23H17NO4 | 372 | 98 | 12,5 | 1 |
| 19 | 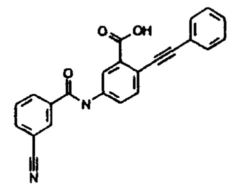 | C23H14N2O3 | 367 | 98 | 12,5 | 1 |
| 20 | 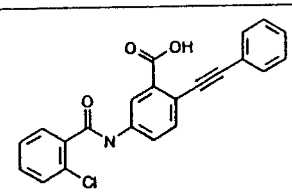 | C22H14ClNO3 | 376 | 100 | 12,5 | 1 |
| 21 | 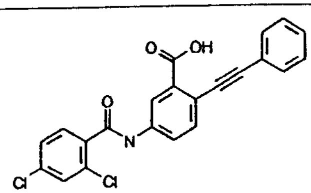 | C22H13Cl2NO3 | 410 | 84 | 12,5 | 1 |
| 22 | 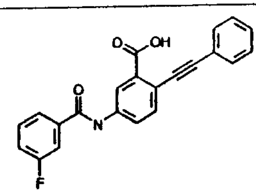 | C22H14FNO3 | 360 | 100 | 12,5 | 1 |
| 23 | 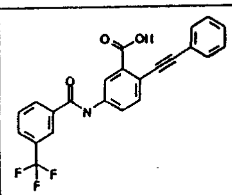 | C23H14F3NO3 | 410 | 63 | 12,5 | 1 |

| | | | | | | |
|---|---|---|---|---|---|---|
| 24 | 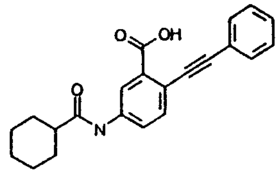 | C22H21NO3 | 348 | 58 | 12,5 | 1 |
| 25 | 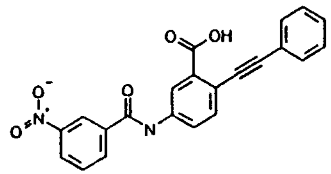 | C22H14N2O5 | 387 | 51 | 12,5 | 1 |
| 26 | 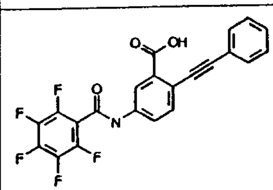 | C22H10F5NO3 | 432 | 54 | 12,5 | 1 |
| 27 | 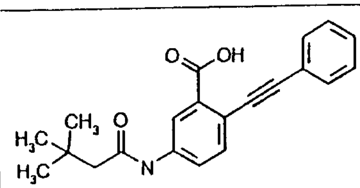 | C21H21NO3 | 336 | 58 | 12,5 | 1 |
| 28 | 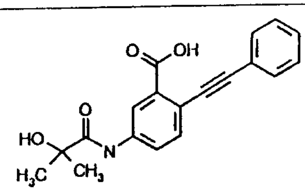 | C19H17NO4 | 324 | 60 | 12,5 | 1 |
| 29 | 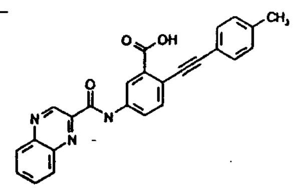 | C25H17N3O3 | 408 | 73 | 12,5 | 1 |

| | | | | | | |
|---|---|---|---|---|---|---|
| 30 | 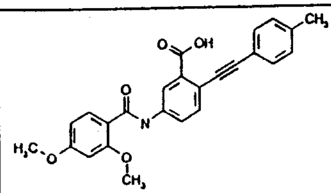 | C25H21NO5 | 416 | 52 | 12,5 | 1 |
| 31 | 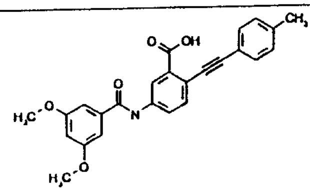 | C25H21NO5 | 416 | 64 | 12,5 | 1 |
| 32 | 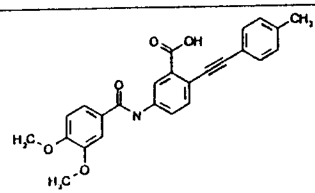 | C25H21NO5 | 416 | 61 | 12,5 | 1 |
| 33 | 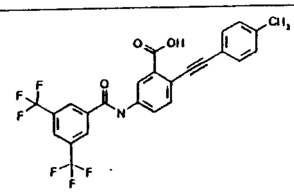 | C25H15F6NO3 | 492 | 77 | 12,5 | 1 |
| 34 | 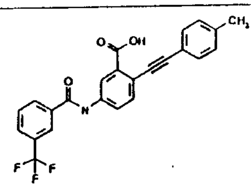 | C24H16F3NO3 | 424 | 50 | 12,5 | 1 |
| 35 | 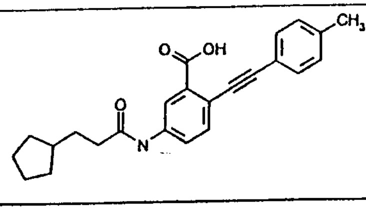 | C24H25NO3 | 376 | 76 | 12,5 | 1 |

| | | | | | | |
|---|---|---|---|---|---|---|
| 36 | 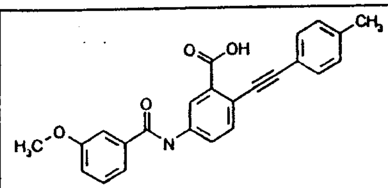 | C24H19NO4 | 386 | 78 | 12,5 | 1 |
| 37 | 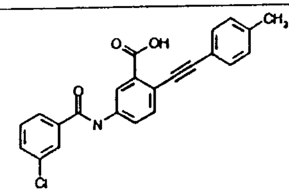 | C23H16ClNO3 | 390 | 55 | 12,5 | 1 |
| 38 | 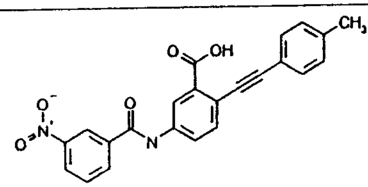 | C23H16N2O5 | 401 | 50 | 12,5 | 1 |
| 39 | 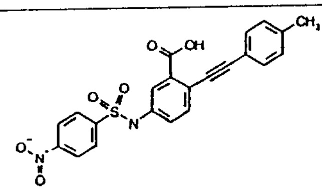 | C22H16N2O6S | 437 | 62 | 12,5 | 1 |
| 40 | 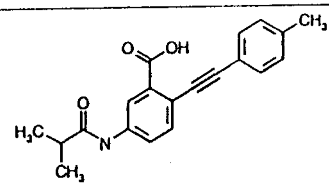 | C20H19NO3 | 322 | 94 | 12,5 | 1 |
| 41 | 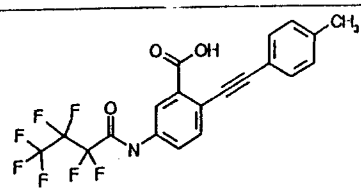 | C20H12F7NO3 | 448 | 71 | 12,5 | 1 |

| | | | | | | |
|---|---|---|---|---|---|---|
| 42 | 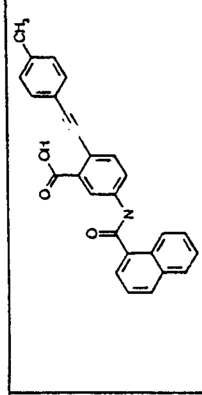 | C27H19NO3 | 406 | 60 | 12,5 | 1 |
| 43 | 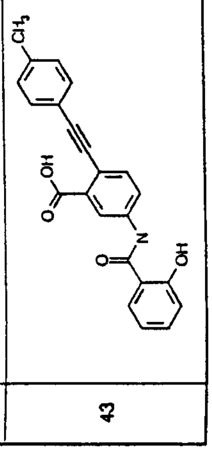 | C23H17NO4 | 372 | 55 | 12,5 | 1 |

Die Herstellung der erfindungsgemäßen Verbindungen der Formel I kann gemäß den in den folgenden Schemata aufgezeigten Verfahren 1 bis 5 erfolgen.

Verfahren 1:

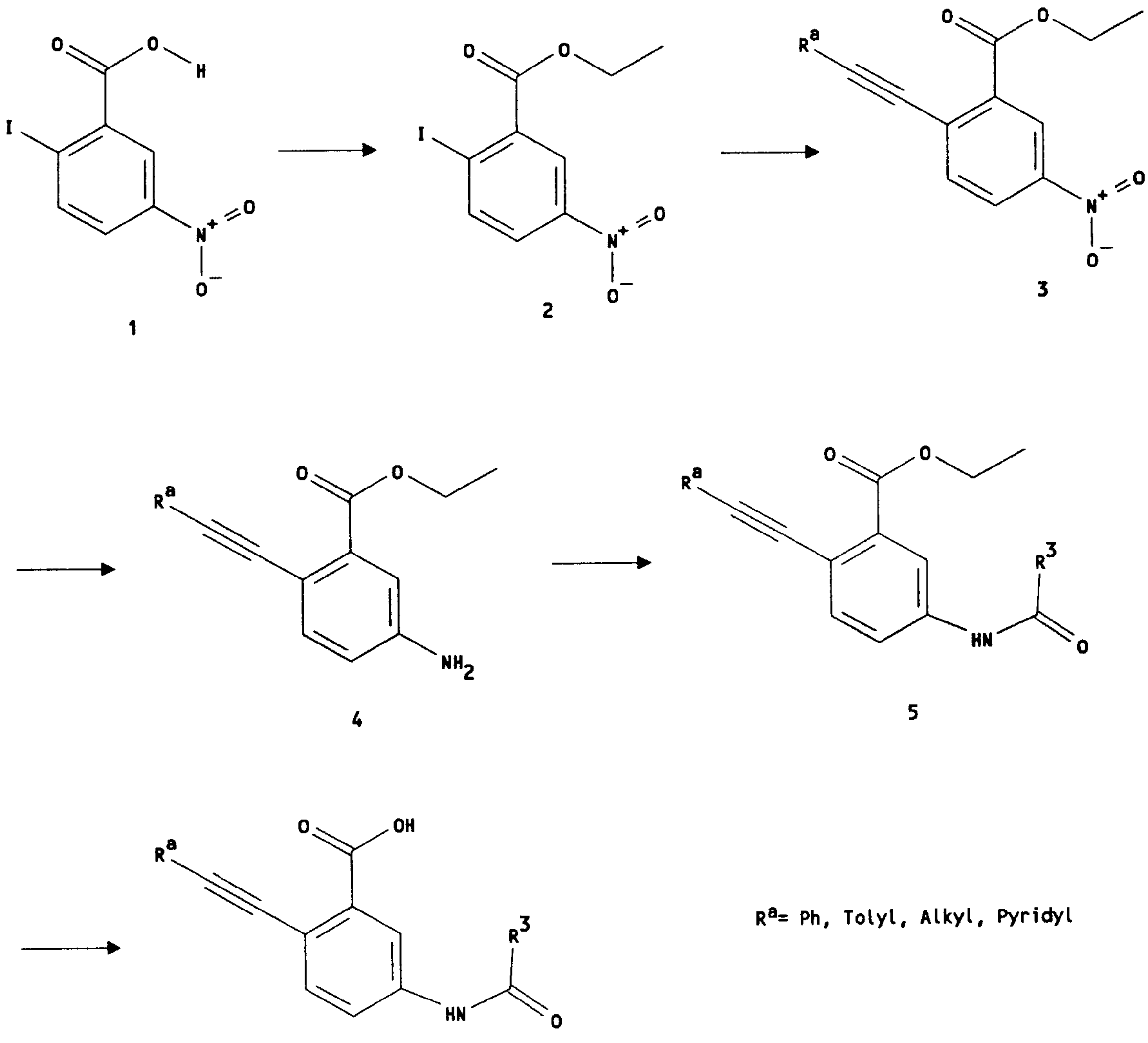

1 2 3 4 5 6

$R^a$= Ph, Tolyl, Alkyl, Pyridyl

Darstellung von 3 aus 1

Die literaturbekannte Verbindung 5-Nitro-2-iodbenzoesäure 1 (Tishchenko, A. D.; Lisitsyn, V. N., Tr. Mosk. Khim.-Tekhnol. Inst. (1967), 52, 126-9.) wurde in Ethanol unter sauerer Katalyse von Thionylchlorid zum entsprechenden Ethylester umgesetzt. Anschließend wurde palladiumkatalysiert der Rest $R^1$ eingeführt.

Hierzu wurden 1 mmol 2 und 2 mmol des entsprechenden terminalen Alkins in einem aprotischen Lösungsmittel wie Toluol, Tetrahydrofuran, Dimethylformamid , bevorzugt Dimethylformamid, in Gegenwart von 3 mmol Triethylamin, 0.15 mmol Kupfer(I)iodid und 0.15 mmol Bistriphenylphosphinpalladium(II)diacetat oder alternativ 0.15 mmol Bistriphenylphosphinpalladium(II)dichlorid bei 10 - 120 °C unter Schutzgas 1 - 12 Stunden erhitzt. Anschließend wurde das Reaktionsgemisch auf gesättigte Ammoniumchlorid-Lösung gegeben und mit Essigester extrahiert. Die abschließende Reinigung erfolgte durch Chromatographie an Kieselgel. Man erhielt 3 in 30 - 80 %iger Ausbeute.

Darstellung von 4 aus 3:

1 mmol 3 wurde in 5 ml Ethanol gelöst und 5 mmol Zinn(II)dichlorid (Hydrat) zugegeben. Nach 4 h bei 40 °C wurde die Reaktionsmischung auf gesättigte Natriumhydrogencarbonat-Lösung gegeben und mit Essigester extrahiert. Das so erhaltene Rohprodukt von 4 wurde ohne weitere Reinigung im Folgeschritt eingesetzt.

Darstellung von 6 aus 4:

1 mmol 4 wurden in 5 ml Dichlormethan gelöst, 3 mmol Triethylamin und bei 0 - 5 °C 2 mmol des ensprechenden Säurechlorids bzw. Isocyanat (kommerziell erhältliche Säurechloride und Isocyanate, bzw. literaturbekannte Säurechloride bzw. Isocyanate) zugegeben. Nach üblicher Aufarbeitung erhielt man in 60 - 90 %iger Ausbeute 5, das durch alkalische Hydrolyse quantitativ in 6 überführt wurde.

Verfahren 2:

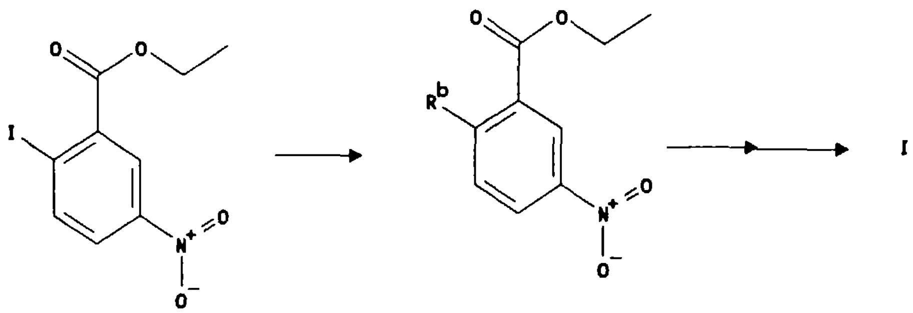

2

7

$R^B$= Aryl, Heteroaryl, 1-Alkeny

Darstellung von 7 aus 2

Zur Einführung von Aryl-, 1-Alkenyl- oder Hetroarylresten wurde 1 mmol 2 in aprotischen Lösungsmitteln wie Dimethylformamid oder Tetrahydrofuran gelöst, 3 Äquivalente Triethylamin und 2 Äquivalente des enstprechenden Aryl-, 1-Alkenyl- oder Heteroaryl-(tributyl)stannans sowie 0,05 Äquivalente Tetrakistriphenyl-hosphinpalladium zugegeben und 50 - 90 °C unter Schutzgas 12 h erhitzt. Anschließend wurde in üblicher Weise aufgearbeitet und 7 durch chomatographische Reinigung an Kieselgel in 60 - 90 %iger Ausbeute erhalten.

Darstellung von I aus 7 erfolgte analog der im Verfahren 1 beschriebenen Umsetzung von 3 nach 6.

Verfahren 3:

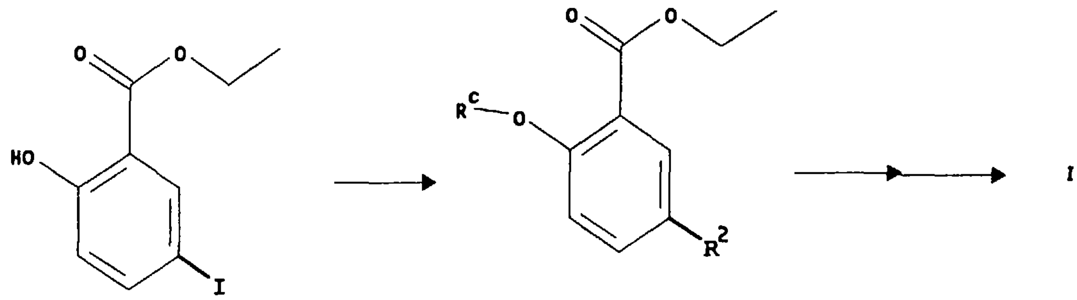

8 9 $R^c$= Benzyl-, $C_1$-$C_6$-Alkyl

Darstellung von 8 aus 2:

Zur Einführung von Benzyl- oder Alkoxy-Resten wurde 1 mmol der literaturbekannten Verbindung 8 (Cacchi, Sandro; Felici, Marcello; Pietroni, Biancarosa., Tetrahedron Lett. (1984), 25(29), 3137 - 40. In einem aprotischen Lösungmittel wie THF, Diethylether oder Acetonitril gelöst, und nacheinander 2 mmol des entsprechenden Benzyl- bzw. aliphatischen Alkohols, 2 mmol Triphenylphosphin sowie tropfenweise Azodicarbonsäurediethylester (alternativ kann auch Azodicarbonsäurediisopropylester oder Azodicarbonsäurebisdimethylamid verwendet werden) zugegeben. Man rührte 4 h bei 20 - 40 °C und erhielt nach üblicher Aufarbeitung und chromatographischer Reinigung an Kieselgel 9 in 70 - 95 %iger Ausbeute.

Darstellung von I aus 9

Darstellung von I aus 9 erfolgte analog der im Verfahren 1 beschriebenen Umsetzung von 3 nach 6.

Verfahren 4

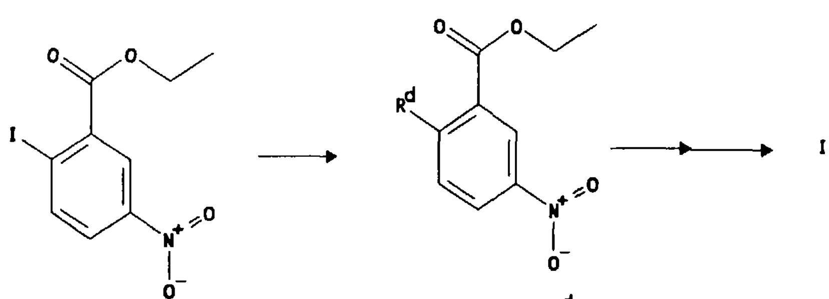

$R^d$= $C_1$-$C_{10}$-Alkyl, $C_1$-$C_4$-Alkyl-(Aryl)$_{1-2}$

2 10

Zur Einführung von Alkyl- bzw Arylalkylresten wurde 1 mmol 2 in einem aprotischen Lösungmittel, wie z. B. DMF

oder Tetrahydrofuran gelöst und unter Schutzgas 2 mmol des entsprechenden Monoalkyl- bzw Monoarylalkylzinkiods (die entsprechenden zinkorganischen Reagentien wurden aus kommerziell erhältlichen Alkyl- bzw. Arylalkyliodiden und aktivierten Zink in Tetrahydrofuran durch 12 stündiges Erhitzen unter Schutzgas hergestellt) und 5 mol % Bistriphenylphosphinpalladiumdiacetat zugegeben. Man erwärmte 4 Stunden auf 60 °C und erhielt nach üblicher Aufarbeitung und chromatographischer Reinigung 10 in 50 - 95 %iger Ausbeute.

Darstellung von I aus 10

Darstellung von I aus 10 erfolgte analog der im Verfahren 1 beschriebenen Umsetzung von 3 nach 6.

Verfahren 5:

Die oben genannten Verfahren lassen sich auch auf Esterderivate der Formel II übertragen, die über einen Linker an ein Polymer, wie z.B. an Polystyrol gebunden sind, sodaß eine Reaktionssequenz ohne Aufarbeitung möglich ist.

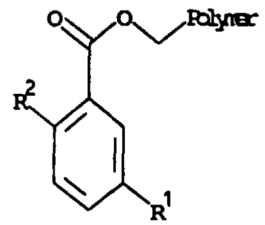

II

Synthesebeispiel für „Festphasensynthese":

A. Synthese der polymer gebundenen 2-Acetoxy-5-iodo-benzoesäure (I)

100 g käufliches Sasrin-Polystyrolharz (Fa. Bachem, Beladung 0.9 mmol/g) werden mit 100ml Dichlormethan versetzt. Man läßt das Harz 30 min bei Raumtempertaur unter vorsichtigem Schütteln quellen. Es wird anschließend mit 137.7 g ( 0.45 mol) 2-Acetoxy-5-iodo-benzoesäure, 56.2 g (0.445 mol) Diisopropylcarbodiimid und 10 mg N,N-Dimethylaminopyridin versetzt. Man läßt über Nacht unter Schütteln stehen. Es wird über eine Fritte abgesaugt und mehrfach mit Dichlormethan, DMF und Ethanol gewaschen.

B. Pd katalysierte C-C Verknüpfung durch 5-Jod Substitution Beispiel: polymer gebundene 2-Acetoxy-5-phenyl-benzoesäure (II)

1 g (I) (0.6 mMol) werden in 10 ml DMF gequollen. Man versetzt mit 12 mg (10 Mol %) Kupferjodid, 24 mg (5 Mol %) Pd(PPh3)2(OAc)2 sowie 840 µl Triethylamin und addiert unter Argon 0,98 ml Tributylzinnphenyl. Es wird 5 h bei 80 °C gerührt. Das Lösungsmittel wird vom Harz abgesaugt, man wäscht gründlich mehrfach mit DMF und Dichlormethan und trochnet das Harz. Eine Probeabspaltung vom Harz mit Trifluoressigsäure (5 %) in Dichlormethan liefert (II) in ca 90 %iger Reinheit (HPLC).

C. Deacetylierung am Harz

Beispiel: Synthese polymer gebundener 2-Hydroxy-5-phenyl-benzoesäure (III)

1 g (II) werden in 25 ml DMF gequollen und mit 5 ml wäßriger 2n NaOH versetzt. Man rührt 6 h bei RT, saugt anschließend vom Lösungsmittel ab und wäscht gründlich mit DMF und Dichlormethan. Eine Probeabspaltung vom Harz wie unter B liefert (III) in ca 90 %iger Reinheit (HPLC).

Nach diesem weiteren Verfahren wurden auch die Beispiele 180 - 289 hergestellt

D. Mitsunobu Reaktion am Harz

Beispiel: Synthese polymer gebundener 2-Benzyloxy- 5-phenylbenzoesäure (IV)

200 mg (III) werden in 2ml THF/1,2-Dichlorethan (1 : 1) gequollen, mit 120 µl Diethylazodicarboxylat in 1 ml THF sowie mit 200 mg Triphenylphosphin in 1 ml 1,2-Dichlorethan und mit 156 µl Benzylalkohol versetzt und 6 h bei 50 °C unter Argon geschüttelt. Man saugt anschließend ab, wäscht mit THF und wiederholt die Reaktion unter identischen Bedingungen. Nach gründlichem Waschen mit THF, DMF und Dichlormethan wird das Harz getrocknet. Eine Probeabspaltung liefert (IV) in ca. 80 %iger Reinheit (HPLC, MS).

E. Trifluormethansulfonierung

Beispiel: Synthese polymer gebundener 2-Trifluormethylsulfonyl-5-phenylbenzoesäure (V)

200 mg (II) werden in 2ml trockenem Dichlormethan gequollen und mit 180 mg N,N-Dimethylaminopyridin versetzt. Man addiert unter Argon bei 0 °C vorsichtig 80 µl Trifluormethansulfonsäureanhydrid gelöst in 0,5 ml trockenem Dichlormethan.

Es wird 30 min bei 0 °C und weitere 30 min bei RT vorsichtig unter Argon geschüttelt. Man wäscht gründlich mit Dichlormethan und DMF. Eine Probeabspaltung vom Harz liefert (V) in ca. 90 %iger Reinheit (HPLC).

F. Pd katalysierte C-C Verknüpfung durch Substitution der 6-Trifluormethylsulfonyl-Einheit

Beispiel: Synthese polymer gebundene 2-Hexin-1-yl-5-phenyl-benzoesäure (VI)

200 mg (V) werden in 2 ml DMF gequollen. Man versetzt mit 3 mg (10 Mol %) Kupferjodid, 6 mg (5 Mol %) Pd(PPh3)2(OAc)2 sowie 210 µl Triethylamin und addiert unter Argon 90 µl 1-Hexin. Es wird 5 h bei 80 °C gerührt. Das Lösungsmittel wird vom Harz abgesaugt, man wäscht gründlich mehrfach mit DMF und Dichlormethan und trochnet das Harz. Eine Probeabspaltung vom Harz mit Trifluoressigsäure (5 %) in Dichlormethan liefert (VI) in ca 80 %iger Reinheit (HPLC,MS).

Ein weiterer Gegenstand der Erfindung sind Arzneimittel, die ein oder mehrere erfindungsgemäße Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen ( = Wirkstoff) entweder als solche, oder vorzugsweise in Kombination mit geegneten pharmazeutischen Hilfsstoffen in Form von Tabletten, Dragees, Kapseln, Suppositorien, Emulsionen, Suspensionen, Granulaten, Pulvern, Lösungen oder Präparate mit protalierter Wirkstoff-Freigabe, eingesetzt, wobei der Wirkstoffgehalt vorteilhafterweise 0,1 bis 95 % beträgt.

Welche Hilfstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidatien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral oder intravenös appliziert werden, wobei die bevorzugte Applikationsart von der zu behandelnden Krankheit abhängig ist. Die orale Verabreichung ist bevorzugt.

Für eine orale Anwendungsform werden die aktiven Verbindungen mit den dafür geeigneten Zusatzstoffen wie Trägerstoffen, Stabilisatoren oder inerten Verdünndungsmittel vermischt und durch üblichen Methoden in geeigneten Dareichungsformen gebracht, wie Tabletten, Dragees, Steckkapseln, wässrige, alkoholische oder ölige Suspensionen oder wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus verschiedenen Lösungsmitteln.

Als pharmazeutische Präparate für topische und lokale Verwendung eignen sich Augentropfen, welche die aktive Verbindung in wässriger oder öliger Lösung enthalten. Für die Anwendung an der Nase sind Aerosole und Sprays, sowie grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Die Dosierung des zu verabreichenden Wirkstoffs der Formel I und die Häufigkeit der Verabreichung hängen von der Wirkstärke und Wirkdauer der verwendeten erfindungsgemäßen Verbindung ab; außerdem auch von der Art und Stärke der zu behandelnden Krankheit sowie von Geschlecht, Alter, Gewicht und individuellen Ansprechbarkeit des zu behandelnden Säugers. Im Durchschnitt liegt die empfohlene tägliche Dosis einer erfindungsgemäßen Verbindung bei einem etwa 75 Kg schweren Säuger - in erster Linier einem Menschen - im Bereich von etwa 10 bis 500 mg, vorzugweise von etwa 25 bis 250 mg, wobei die Verabreichung nach Bedarf in mehreren Dosen pro Tag erfolgen kann.

Die Beispiele 1 - 43 aus Tabelle 1 sowie die nachfolgenden Beispiele 44 - 352 aus Tabelle 2 sollen die vorliegende Erfindung illustrieren, ohne sie jedoch in ihren Umfang einzuschränken.

Tabelle 2:

| Nr. | Struktur | Summenformel | MS (M+H+) | Herstellungsverfahren |
|---|---|---|---|---|
| 44 | 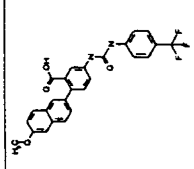 | C26H19F3N2O4 | 481 | 1 |
| 45 | 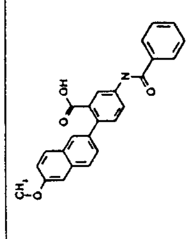 | C25H19NO4 | 398 | 2 |
| 46 | 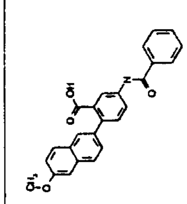 | C25H19NO4 | 398 | 2 |
| 47 | 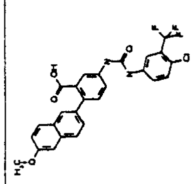 | C26H18ClF3N2O4 | 515 | 2 |
| 48 | 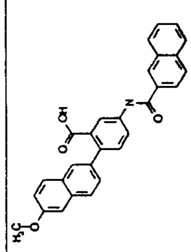 | C29H21NO4 | 448 | 2 |

| | | | | |
|---|---|---|---|---|
| 49 | 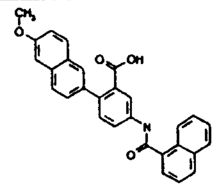 | C29H21NO4 | 448 | 2 |
| 50 | 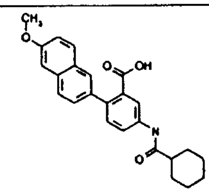 | C25H25NO4 | 404 | 2 |
| 51 | 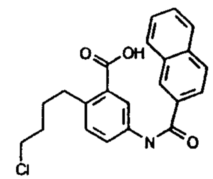 | C22H20ClNO3 | 382 | 4 |
| 52 | 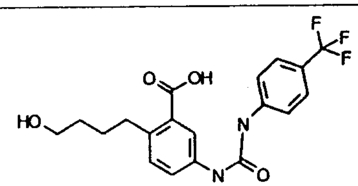 | C19H19F3N2O4 | 397 | 5 |
| 53 | 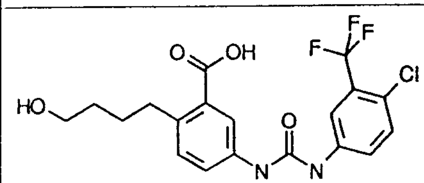 | C19H18ClF3N2O4 | 431 | 5 |
| 54 | 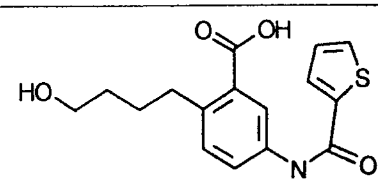 | C16H17NO4S | 320 | 4 |

| | | | | |
|---|---|---|---|---|
| 55 | 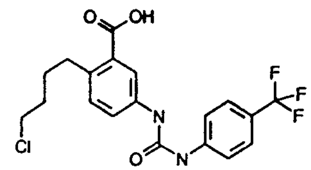 | C19H18ClF3N2O3 | 415 | 4 |
| 56 | 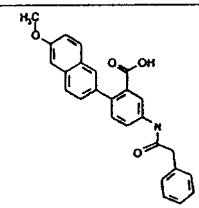 | C26H21NO4 | 412 | 2 |
| 57 | 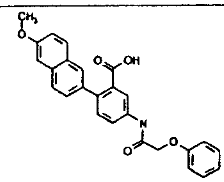 | C26H21NO5 | 428 | 2 |
| 58 | 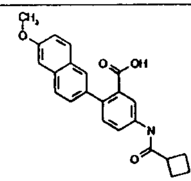 | C23H21NO4 | 376 | 2 |
| 59 | 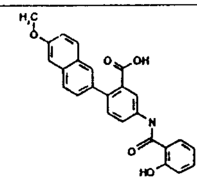 | C25H19NO5 | 414 | 2 |
| 60 | 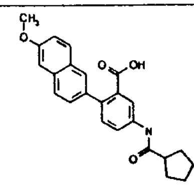 | C24H23NO4 | 390 | 2 |

| | | | | |
|---|---|---|---|---|
| 61 | 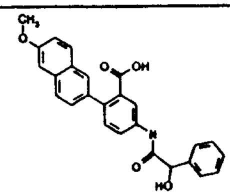 | C26H21NO5 | 428 | 2 |
| 62 | 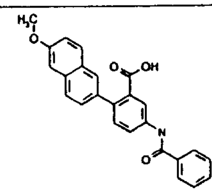 | C25H19NO4 | 398 | 2 |
| 63 | 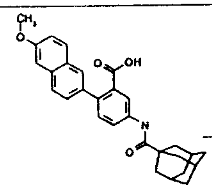 | C29H29NO4 | 456 | 2 |
| 64 | 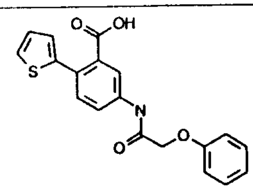 | C19H15NO4S | 354 | 2 |
| 65 | 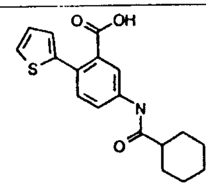 | C18H19NO3S | 330 | 2 |
| 66 | 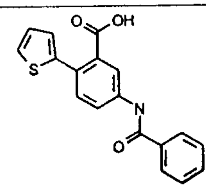 | C18H13NO3S | 324 | 2 |

| | | | | |
|---|---|---|---|---|
| 67 | 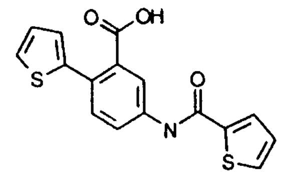 | C16H11NO3S2 | 330 | 2 |
| 68 | 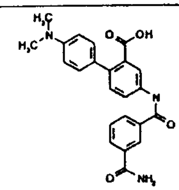 | C23H21N3O4 | 404 | 2 |
| 69 | 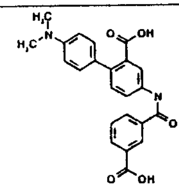 | C23H20N2O5 | 405 | 2 |
| 70 | 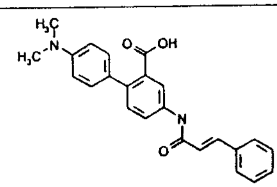 | C24H22N2O3 | 387 | 2 |
| 71 | 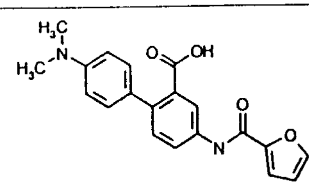 | C20H18N2O4 | 351 | 2 |
| 72 | 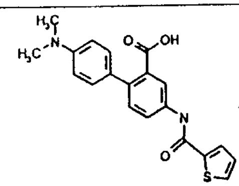 | C20H18N2O3S | 367 | 2 |

| | | | | |
|---|---|---|---|---|
| 73 | 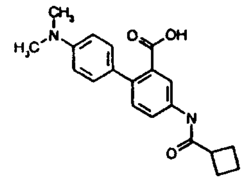 | C20H22N2O3 | 339 | 2 |
| 74 | 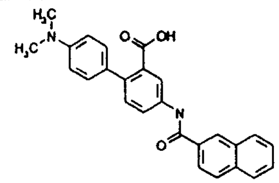 | C26H22N2O3 | 411 | 2 |
| 75 | 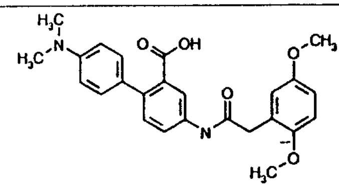 | C25H26N2O5 | 435 | 2 |
| 76 | 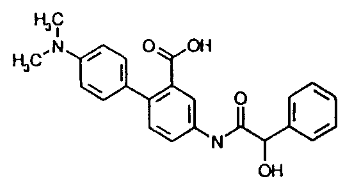 | C23H22N2O4 | 391 | 2 |
| 77 | 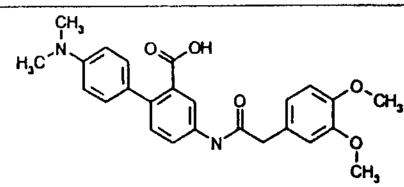 | C25H26N2O5 | 435 | 2 |
| 78 | 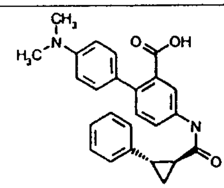 | C25H24N2O3 | 401 | 2 |

| | | | | |
|---|---|---|---|---|
| 79 | 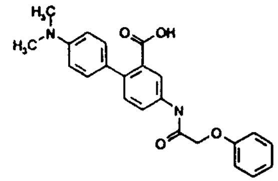 | C23H22N2O4 | 391 | 2 |
| 80 | 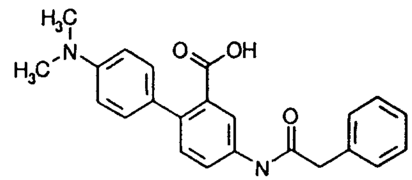 | C23H22N2O3 | 375 | 2 |
| 81 | 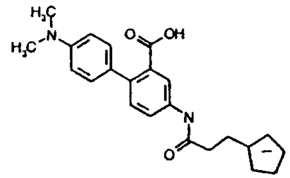 | C23H28N2O3 | 381 | 2 |
| 82 | 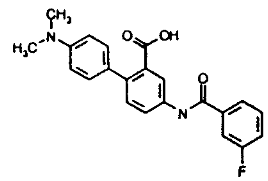 | C22H19FN2O3 | 379 | 2 |
| 83 | 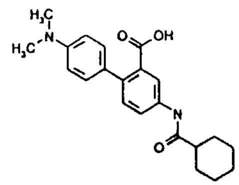 | C22H26N2O3 | 367 | 2 |
| 84 | 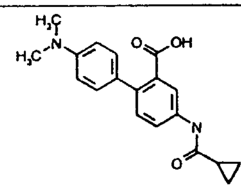 | C19H20N2O3 | 325 | 2 |

| | | | | |
|---|---|---|---|---|
| 85 | 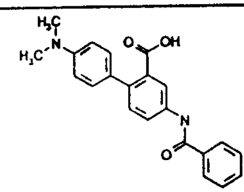 | C22H20N2O3 | 361 | 2 |
| 86 | 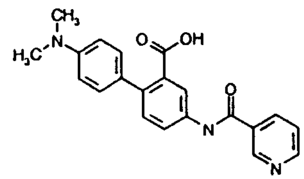 | C21H19N3O3 | 362 | 2 |
| 87 | 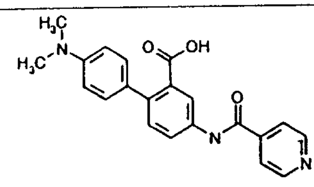 | C21H19N3O3 | 362 | 2 |
| 88 | 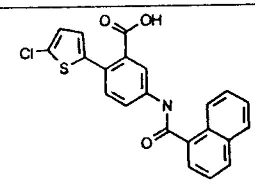 | C22H14ClNO3S | 408 | 2 |
| 89 | 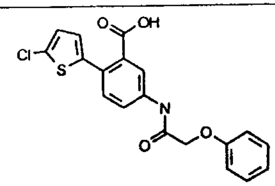 | C19H14ClNO4S | 389 | 2 |
| 90 | 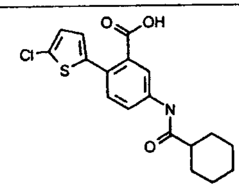 | C18H18ClNO3S | 364 | 2 |

| | | | | |
|---|---|---|---|---|
| 91 | 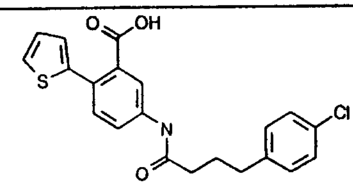 | C21H18ClNO3S | 400 | 2 |
| 92 | 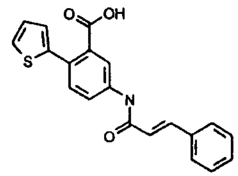 | C20H15NO3S | 350 | 2 |
| 93 | 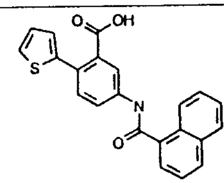 | C22H15NO3S | 374 | 2 |
| 94 | 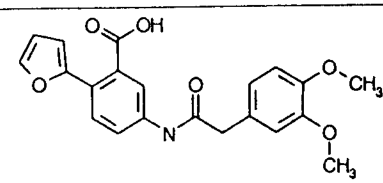 | C21H19NO6 | 382 | 2 |
| 96 | 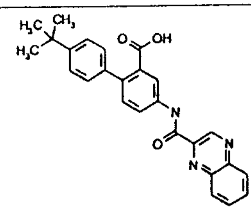 | C26H23N3O3 | 426 | 2 |
| 98 | 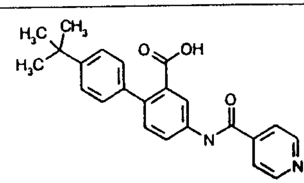 | C23H22N2O3 | 375 | 2 |

| | | | | |
|---|---|---|---|---|
| 101 | 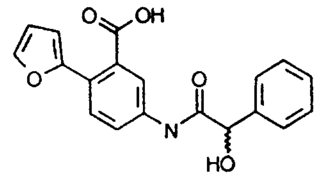 | C19H15NO5 | 338 | 2 |
| 102 | 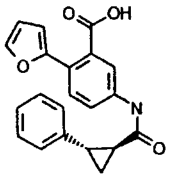 | C21H17NO4 | 348 | 2 |
| 103 | 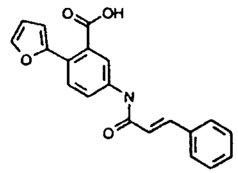 | C20H15NO4 | 334 | 2 |
| 104 | 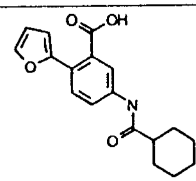 | C18H19NO4 | 314 | 2 |
| 105 | 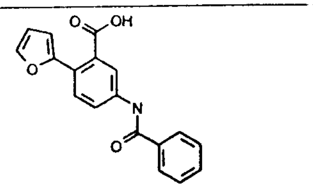 | C18H13NO4 | 308 | 2 |
| 106 | 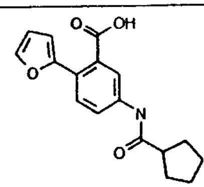 | C17H17NO4 | 300 | 2 |

| | | | | |
|---|---|---|---|---|
| 107 | 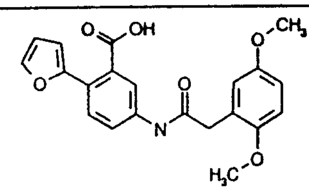 | C21H19NO6 | 382 | 2 |
| 108 | 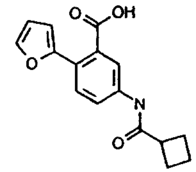 | C16H15NO4 | 286 | 2 |
| 109 | 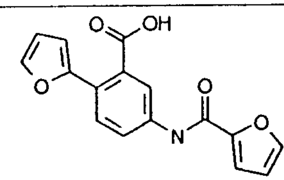 | C16H11NO5 | 298 | 2 |
| 110 | 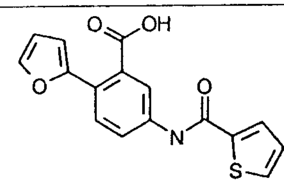 | C16H11NO4S | 314 | 2 |
| 111 | 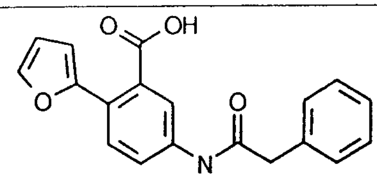 | C19H15NO4 | 322 | 2 |
| 112 | 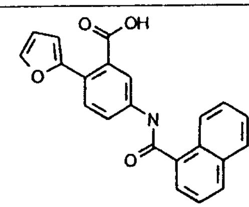 | C22H15NO4 | 358 | 2 |

| | | | | |
|---|---|---|---|---|
| 113 | 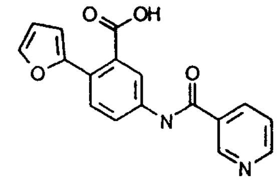 | C17H12N2O4 | 309 | 2 |
| 114 | 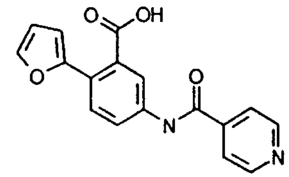 | C17H12N2O4 | 309 | 2 |
| 115 | 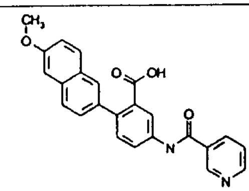 | C24H18N2O4 | 399 | 2 |
| 116 | 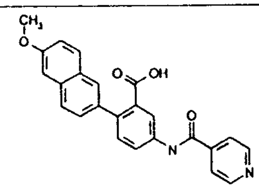 | C24H18N2O4 | 399 | 2 |
| 117 | 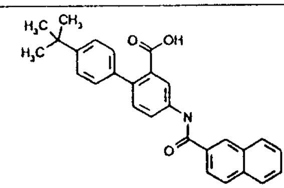 | C28H25NO3 | 424 | 2 |
| 118 | 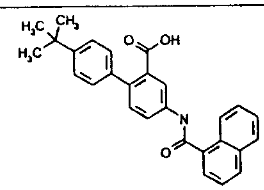 | C28H25NO3 | 424 | 2 |

| | | | | |
|---|---|---|---|---|
| 119 | 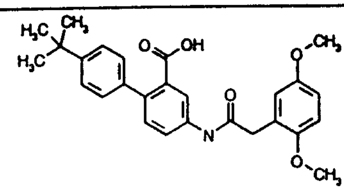 | C27H29NO5 | 448 | 2 |
| 120 | 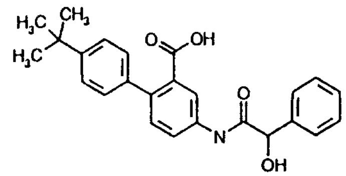 | C25H25NO4 | 404 | 2 |
| 121 | 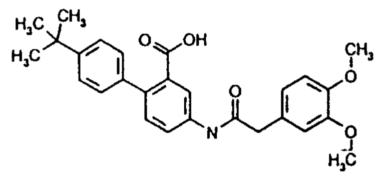 | C27H29NO5 | 448 | 2 |
| 122 | 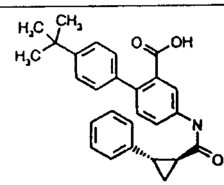 | C27H27NO3 | 414 | 2 |
| 123 | 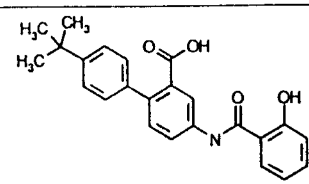 | C24H23NO4 | 390 | 2 |
| 124 | 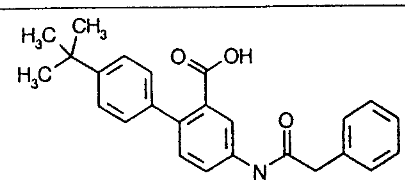 | C25H25NO3 | 388 | 2 |

| | | | | |
|---|---|---|---|---|
| 125 | 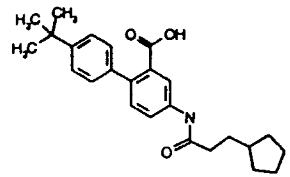 | C25H31NO3 | 394 | 2 |
| 126 | 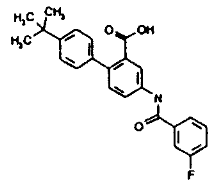 | C24H22FNO3 | 392 | 2 |
| 127 | 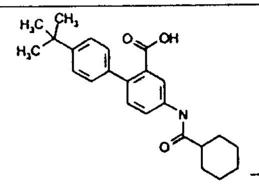 | C24H29NO3 | 380 | 2 |
| 128 | 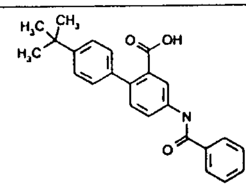 | C24H23NO3 | 374 | 2 |
| 129 | 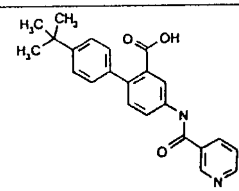 | C23H22N2O3 | 375 | 2 |
| 130 | 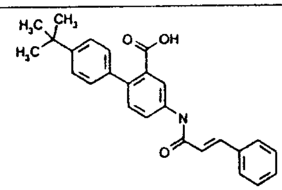 | C26H25NO3 | 400 | 2 |

| | | | | |
|---|---|---|---|---|
| 131 | 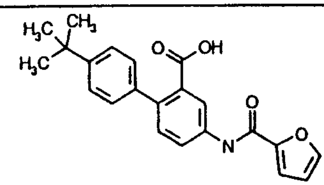 | C22H21NO4 | 364 | 2 |
| 132 | 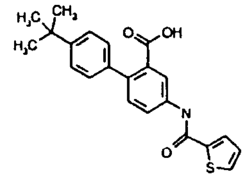 | C22H21NO3S | 380 | 2 |
| 133 | 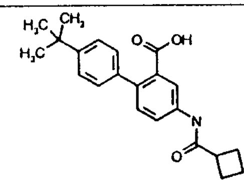 | C22H25NO3 | 352 | 2 |
| 134 | 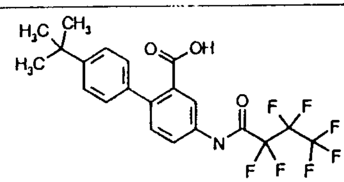 | C21H18F7NO3 | 466 | 2 |
| 135 | 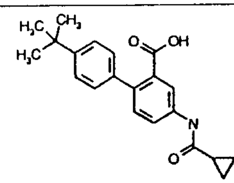 | C21H23NO3 | 338 | 2 |
| 136 | 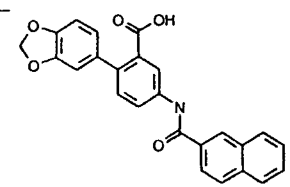 | C25H17NO5 | 412 | 2 |

| | | | | |
|---|---|---|---|---|
| 137 | 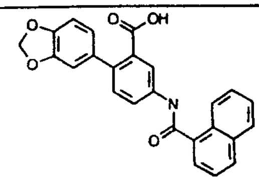 | C25H17NO5 | 412 | 2 |
| 138 | 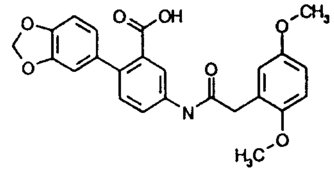 | C24H21NO7 | 436 | 2 |
| 139 | 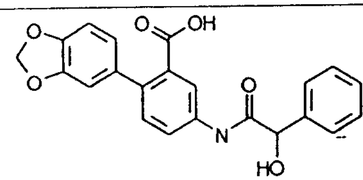 | C22H17NO6 | 392 | 2 |
| 140 | 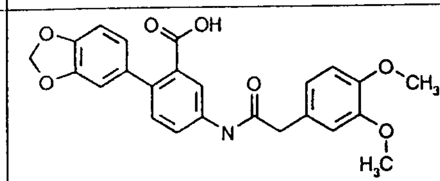 | C24H21NO7 | 436 | 2 |
| 141 | 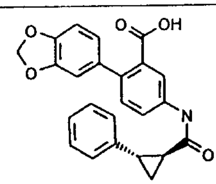 | C24H19NO5 | 402 | 2 |
| 142 | 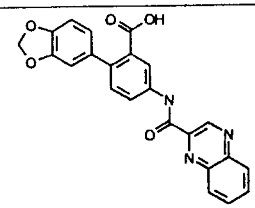 | C23H15N3O5 | 414 | 2 |

| | | | | |
|---|---|---|---|---|
| 143 | 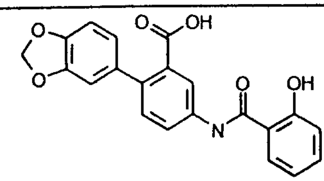 | C21H15NO6 | 378 | 2 |
| 144 | 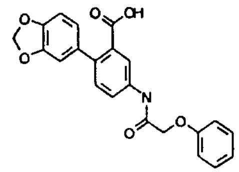 | C22H17NO6 | 392 | 2 |
| 145 | 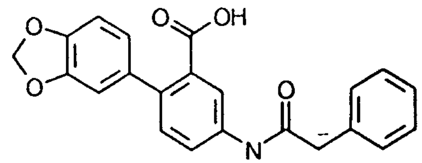 | C22H17NO5 | 376 | 2 |
| 146 | 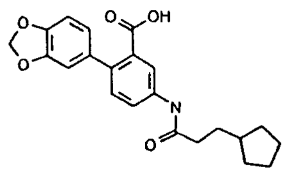 | C22H23NO5 | 382 | 2 |
| 147 | 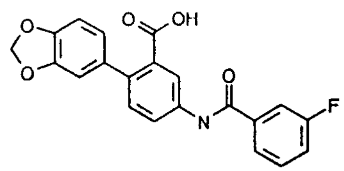 | C21H14FNO5 | 380 | 2 |
| 148 | 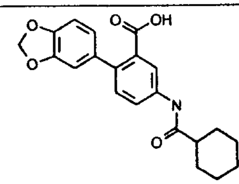 | C21H21NO5 | 368 | 2 |

| | | | | |
|---|---|---|---|---|
| 149 | 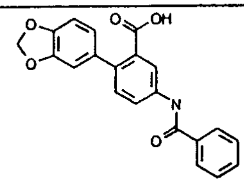 | C21H15NO5 | 362 | 2 |
| 150 | 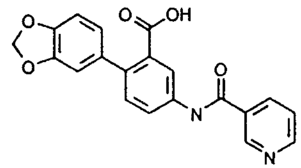 | C20H14N2O5 | 363 | 2 |
| 151 | 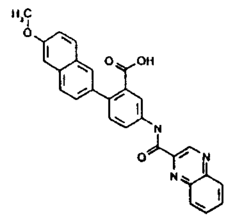 | C27H19N3O4 | 450 | 2 |
| 152 | 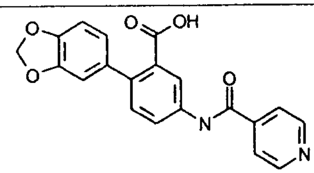 | C20H14N2O5 | 363 | 2 |
| 153 | 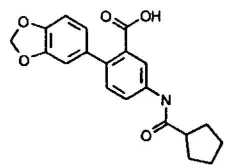 | C20H19NO5 | 354 | 2 |
| 154 | 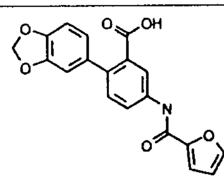 | C19H13NO6 | 352 | 2 |

| | | | | |
|---|---|---|---|---|
| 155 | 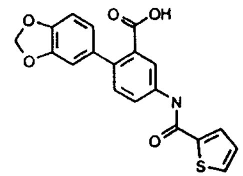 | C19H13NO5S | 368 | 2 |
| 156 | 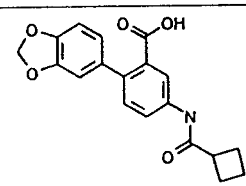 | C19H17NO5 | 340 | 2 |
| 157 | 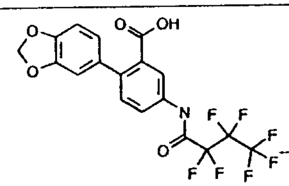 | C18H10F7NO5 | 454 | 2 |
| 158 | 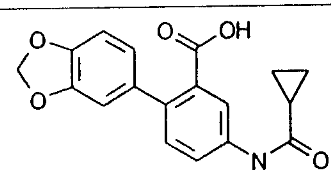 | C18H15NO5 | 326 | 2 |
| 159 | 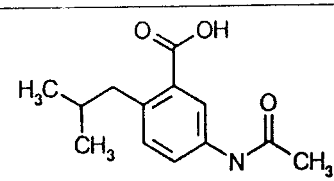 | C13H17NO3 | 236 | 4 |
| 160 | 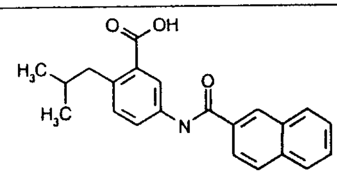 | C22H21NO3 | 348 | 4 |

| | | | | |
|---|---|---|---|---|
| 161 | 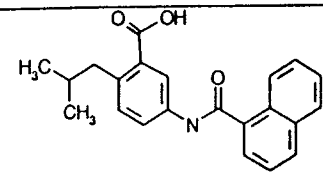 | C22H21NO3 | 348 | 4 |
| 162 | 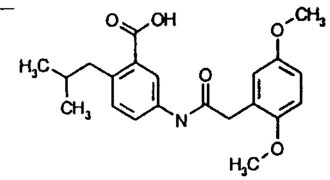 | C21H25NO5 | 372 | 4 |
| 163 | 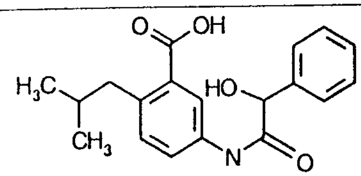 | C19H21NO4 | 328 | 4 |
| 164 | 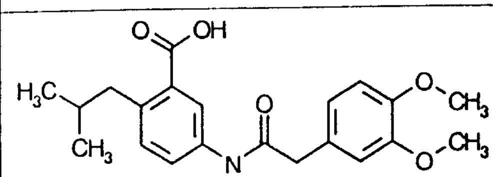 | C21H25NO5 | 372 | 4 |
| 165 | 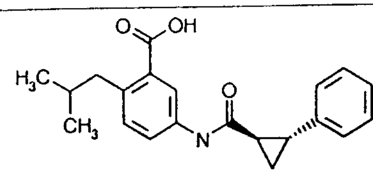 | C21H23NO3 | 338 | 4 |
| 166 | 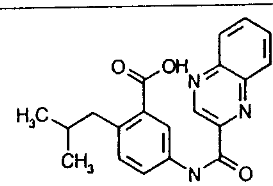 | C20H19N3O3 | 350 | 4 |

| | | | | |
|---|---|---|---|---|
| 167 | 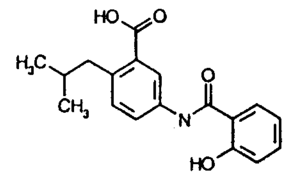 | C18H19NO4 | 314 | 4 |
| 168 | 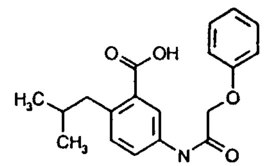 | C19H21NO4 | 328 | 4 |
| 169 | 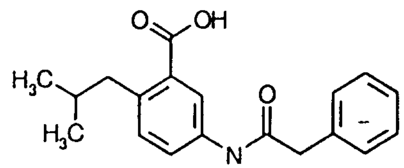 | C19H21NO3 | 312 | 4 |
| 170 | 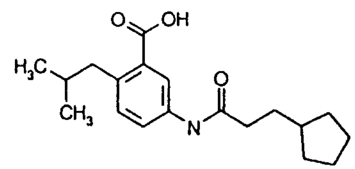 | C19H27NO3 | 318 | 4 |
| 171 | 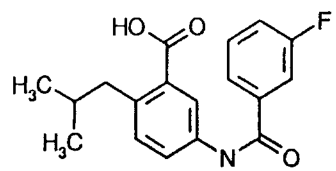 | C18H18FNO3 | 316 | 4 |
| 172 | 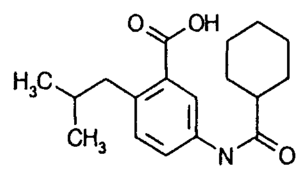 | C18H25NO3 | 304 | 4 |

| | | | | |
|---|---|---|---|---|
| 173 | 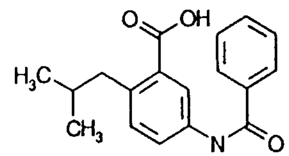 | C18H19NO3 | 298 | 4 |
| 174 | 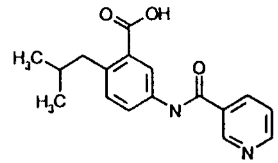 | C17H18N2O3 | 299 | 4 |
| 175 | 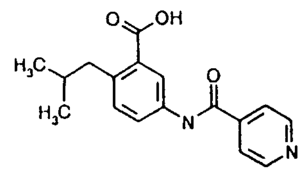 | C17H18N2O3 | 299 | 4 |
| 176 | 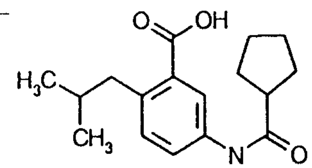 | C17H23NO3 | 290 | 4 |
| 177 | 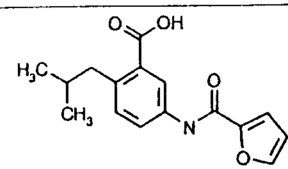 | C16H17NO4 | 288 | 4 |
| 178 | 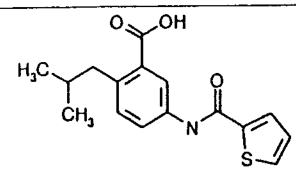 | C16H17NO3S | 304 | 4 |

| | | | | |
|---|---|---|---|---|
| 179 | 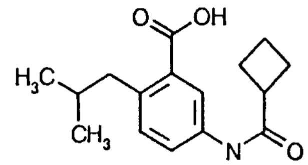 | C16H21NO3 | 276 | 4 |
| 180 | 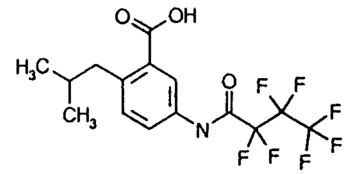 | C15H14F7NO3 | 390 | 4 |
| 181 | 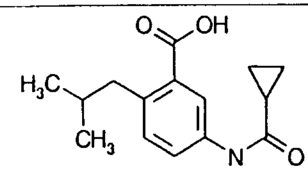 | C15H19NO3 | 262 | 4 |
| 182 | 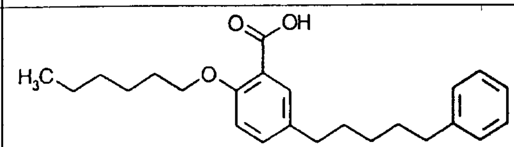 | C24H32O3 | 369 | 3 |
| 183 | 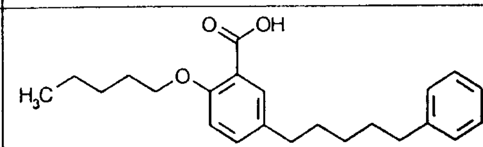 | C23H30O3 | 355 | 3 |
| 184 | 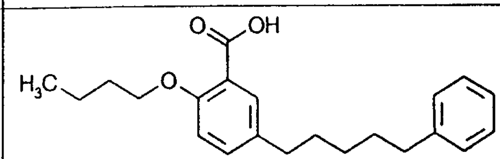 | C22H28O3 | 341 | 3 |

| | | | | |
|---|---|---|---|---|
| 185 | 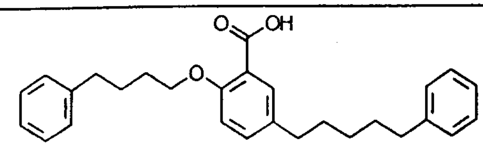 | C28H32O3 | 417 | 3 |
| 186 | 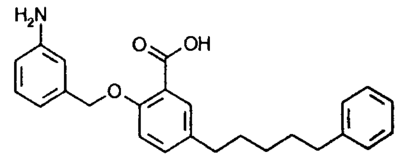 | C25H27NO3 | 390 | 3 |
| 187 | 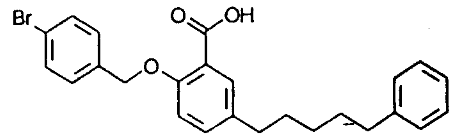 | C25H25BrO3 | 454 | 3 |
| 188 | 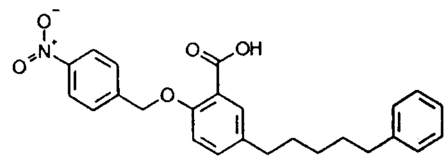 | C25H25NO5 | 420 | 3 |
| 189 | 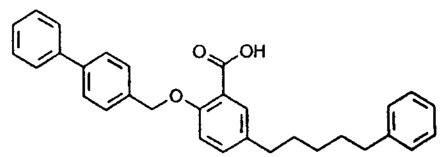 | C31H30O3 | 451 | 3 |
| 190 | 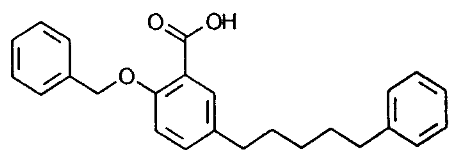 | C25H26O3 | 375 | 3 |

| | | | | |
|---|---|---|---|---|
| 191 | 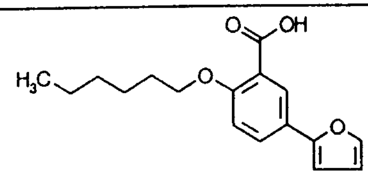 | C17H20O4 | 289 | 3 |
| 192 | 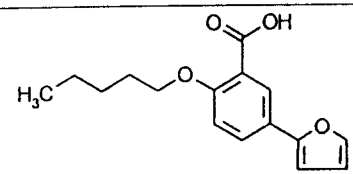 | C16H18O4 | 275 | 3 |
| 193 | 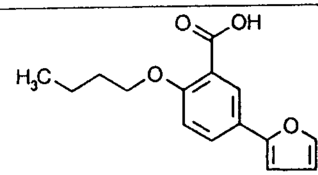 | C15H16O4 | 261 | 3 |
| 194 | 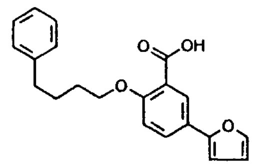 | C21H20O4 | 337 | 3 |
| 195 | 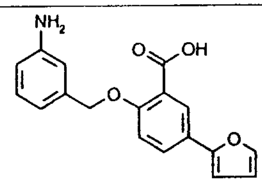 | C18H15NO4 | 310 | 3 |
| 196 | 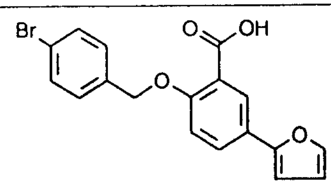 | C18H13BrO4 | 374 | 3 |

| | | | | |
|---|---|---|---|---|
| 197 | 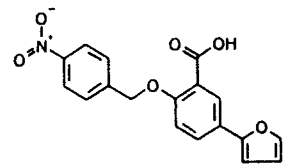 | C18H13NO6 | 340 | 3 |
| 198 | 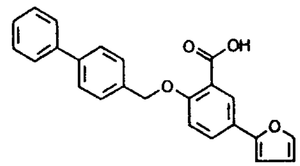 | C24H18O4 | 371 | 3 |
| 199 | 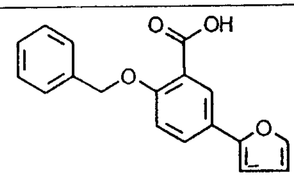 | C18H14O4 | 295 | 3 |
| 200 | 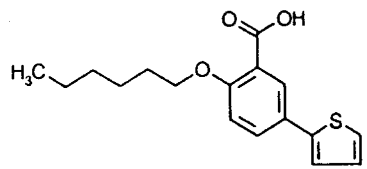 | C17H20O3S | 305 | 3 |
| 201 | 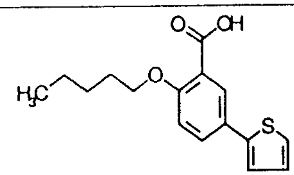 | C16H18O3S | 291 | 3 |
| 202 | 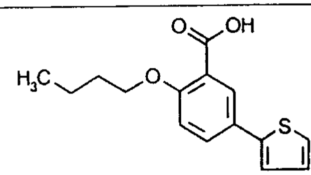 | C15H16O3S | 277 | 3 |

| | | | | |
|---|---|---|---|---|
| 203 | 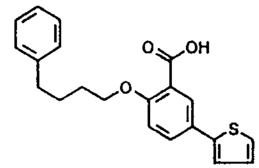 | C21H20O3S | 353 | 3 |
| 204 | 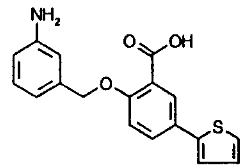 | C18H15NO3S | 326 | 3 |
| 205 | 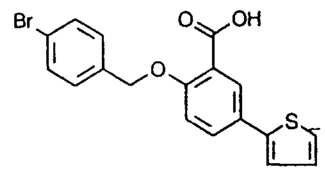 | C18H13BrO3S | 390 | 3 |
| 206 | 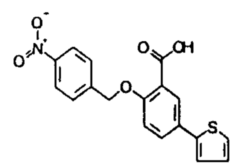 | C18H13NO5S | 356 | 3 |
| 207 | 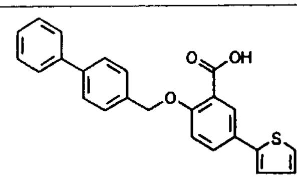 | C24H18O3S | 387 | 3 |
| 208 | 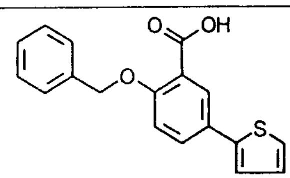 | C18H14O3S | 311 | 3 |

| | | | | |
|---|---|---|---|---|
| 209 | 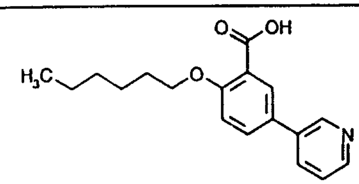 | C18H21NO3 | 300 | 3 |
| 210 | 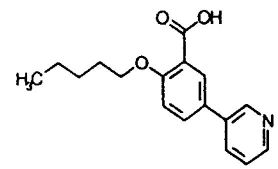 | C17H19NO3 | 286 | 3 |
| 211 | 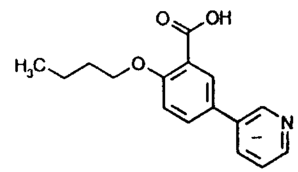 | C16H17NO3 | 272 | 3 |
| 212 | 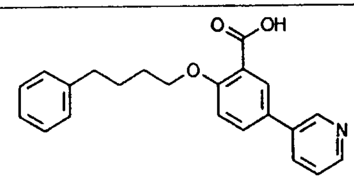 | C22H21NO3 | 348 | 3 |
| 213 | 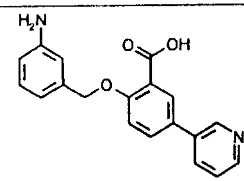 | C19H16N2O3 | 321 | 3 |
| 214 | 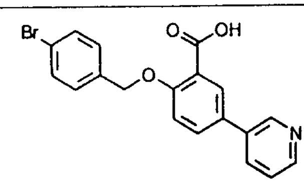 | C19H14BrNO3 | 385 | 3 |

| | | | | |
|---|---|---|---|---|
| 215 | 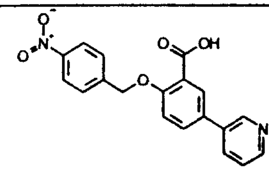 | C19H14N2O5 | 351 | 3 |
| 216 | 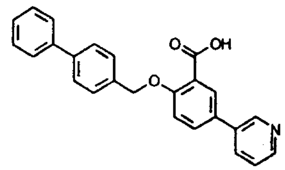 | C25H19NO3 | 382 | 3 |
| 217 | 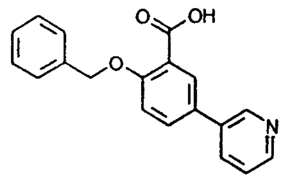 | C19H15NO3 | 306 | 3 |
| 218 | 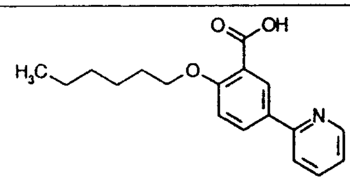 | C18H21NO3 | 300 | 3 |
| 219 | 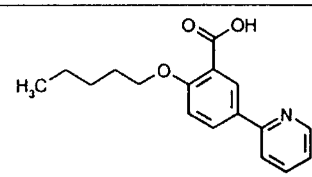 | C17H19NO3 | 286 | 3 |
| 220 | 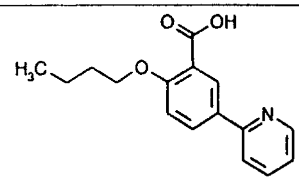 | C16H17NO3 | 272 | 3 |

| | | | | |
|---|---|---|---|---|
| 221 | 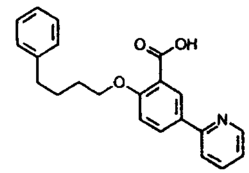 | C22H21NO3 | 348 | 3 |
| 222 | 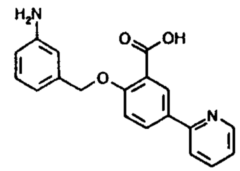 | C19H16N2O3 | 321 | 3 |
| 223 | 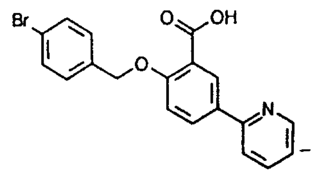 | C19H14BrNO3 | 385 | 3 |
| 224 | 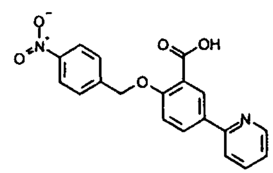 | C19H14N2O5 | 351 | 3 |
| 225 | 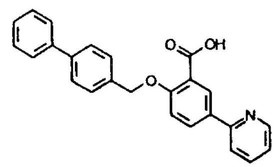 | C25H19NO3 | 382 | 3 |
| 226 | 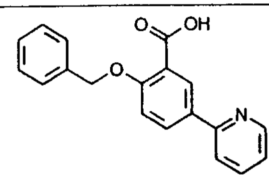 | C19H15NO3 | 306 | 3 |

| | | | | |
|---|---|---|---|---|
| 227 | 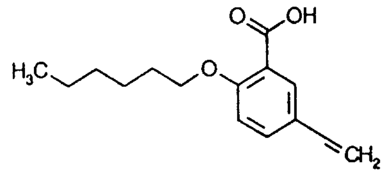 | C15H20O3 | 249 | 3 |
| 228 | 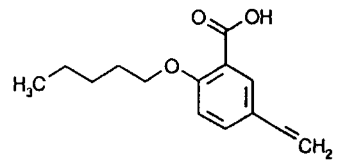 | C14H18O3 | 235 | 3 |
| 229 | 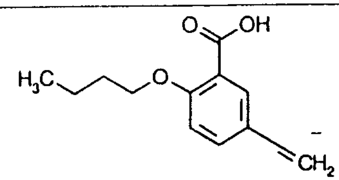 | C13H16O3 | 221 | 3 |
| 230 | 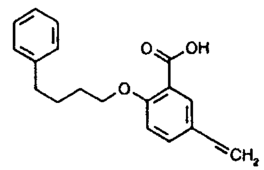 | C19H20O3 | 297 | 3 |
| 231 | 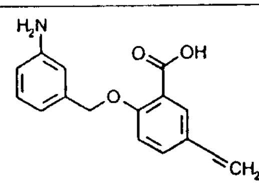 | C16H15NO3 | 270 | 3 |
| 232 | 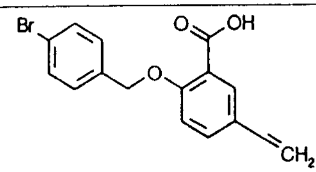 | C16H13BrO3 | 334 | 3 |

| | | | | |
|---|---|---|---|---|
| 233 | 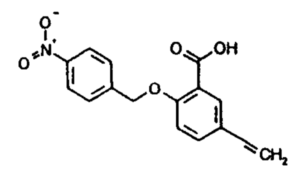 | C16H13NO5 | 300 | 3 |
| 234 | 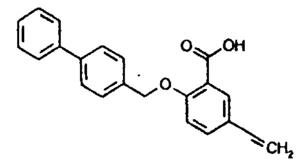 | C22H18O3 | 331 | 3 |
| 235 | 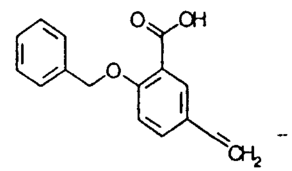 | C16H14O3 | 255 | 3 |
| 236 | 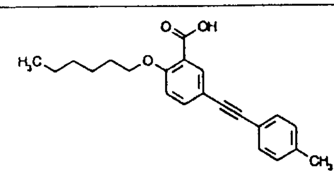 | C22H24O3 | 337 | 3 |
| 237 | 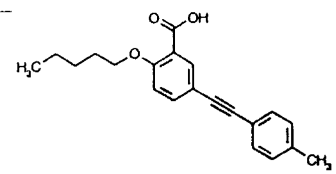 | C21H22O3 | 323 | 3 |
| 238 | 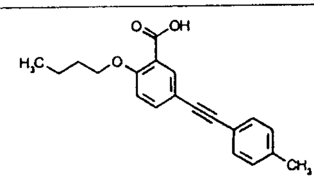 | C20H20O3 | 309 | 3 |

| | | | | |
|---|---|---|---|---|
| 239 | 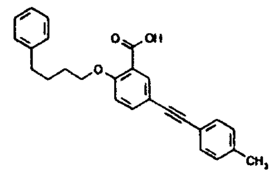 | C26H24O3 | 385 | 3 |
| 240 | 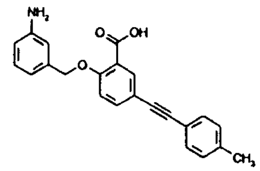 | C23H19NO3 | 358 | 3 |
| 241 | 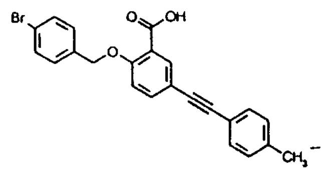 | C23H17BrO3 | 422 | 3 |
| 242 | 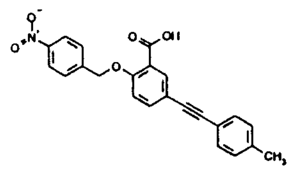 | C23H17NO5 | 388 | 3 |
| 243 | 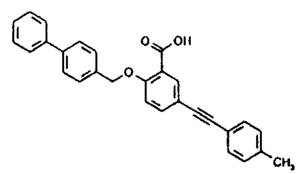 | C29H22O3 | 419 | 3 |
| 244 | 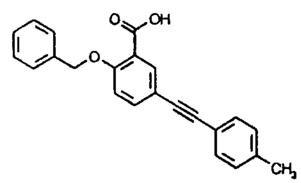 | C23H18O3 | 343 | 3 |

| | | | | |
|---|---|---|---|---|
| 245 | 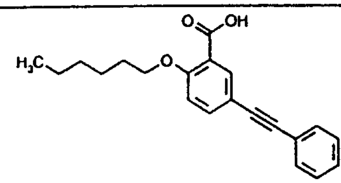 | C21H22O3 | 323 | 3 |
| 246 | 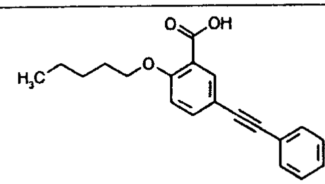 | C20H20O3 | 309 | 3 |
| 247 | 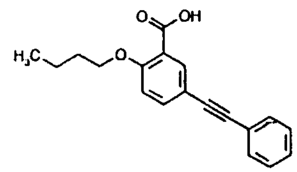 | C19H18O3 | 295 | 3 |
| 248 | 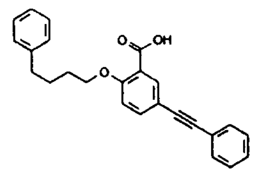 | C25H22O3 | 371 | 3 |
| 249 | 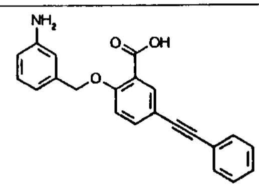 | C22H17NO3 | 344 | 3 |
| 250 | 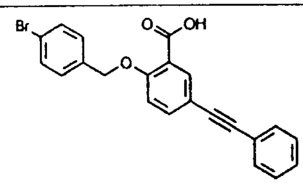 | C22H15BrO3 | 408 | 3 |

| | | | | |
|---|---|---|---|---|
| 251 | 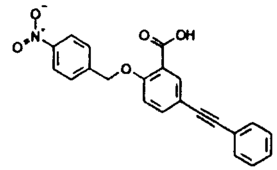 | C22H15NO5 | 374 | 3 |
| 252 | 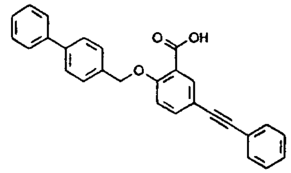 | C28H20O3 | 405 | 3 |
| 253 | 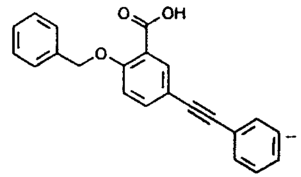 | C22H16O3 | 329 | 3 |
| 254 | 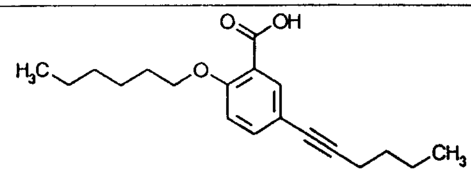 | C19H26O3 | 303 | 3 |
| 255 | 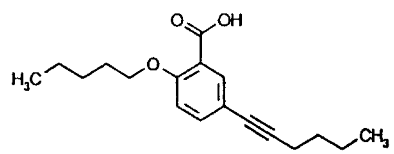 | C18H24O3 | 289 | 3 |
| 256 | 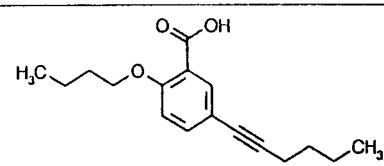 | C17H22O3 | 275 | 3 |

| | | | | |
|---|---|---|---|---|
| 257 | 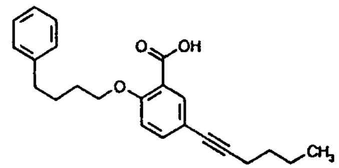 | C23H26O3 | 351 | 3 |
| 258 | 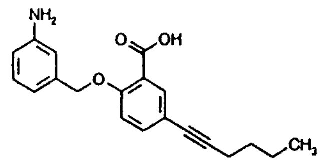 | C20H21NO3 | 324 | 3 |
| 259 | 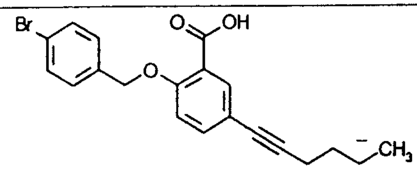 | C20H19BrO3 | 388 | 3 |
| 260 | 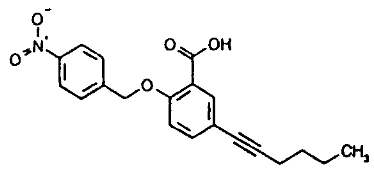 | C20H19NO5 | 354 | 3 |
| 261 | 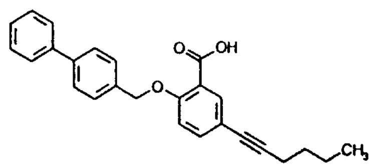 | C26H24O3 | 385 | 3 |
| 262 | 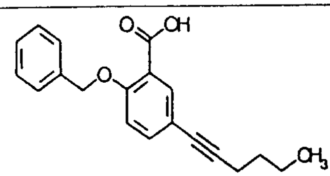 | C20H20O3 | 309 | 3 |

| | | | | |
|---|---|---|---|---|
| 263 | 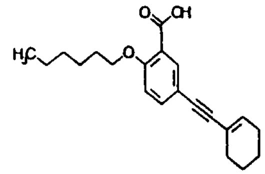 | C21H26O3 | 327 | 3 |
| 264 | 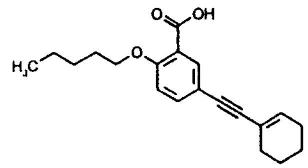 | C20H24O3 | 313 | 3 |
| 265 | 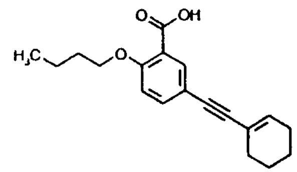 | C19H22O3 | 299 | 3 |
| 266 | 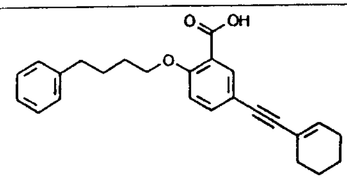 | C25H26O3 | 375 | 3 |
| 267 | 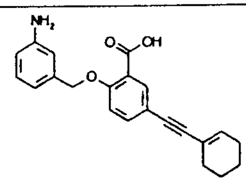 | C22H21NO3 | 348 | 3 |
| 268 | 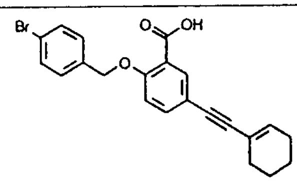 | C22H19BrO3 | 412 | 3 |

| | | | | |
|---|---|---|---|---|
| 269 | 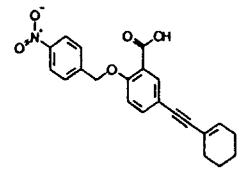 | C22H19NO5 | 378 | 3 |
| 270 | 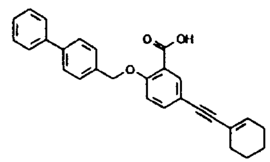 | C28H24O3 | 409 | 3 |
| 271 | 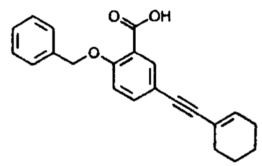 | C22H20O3 | 333 | 3 |
| 272 | 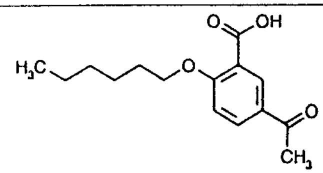 | C15H20O4 | 265 | 3 |
| 273 | 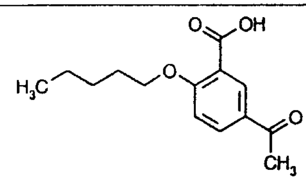 | C14H18O4 | 251 | 3 |
| 274 | 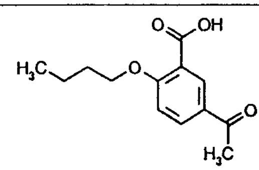 | C13H16O4 | 237 | 3 |

| | | | | |
|---|---|---|---|---|
| 275 | 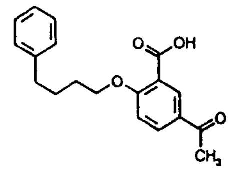 | C19H20O4 | 313 | 3 |
| 276 | 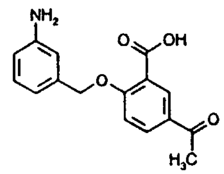 | C16H15NO4 | 286 | 3 |
| 277 | 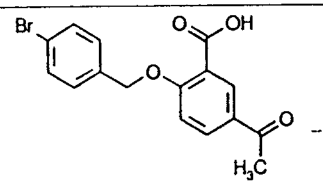 | C16H13BrO4 | 350 | 3 |
| 278 | 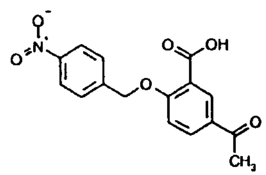 | C16H13NO6 | 316 | 3 |
| 279 | 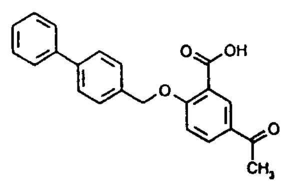 | C22H18O4 | 347 | 3 |
| 280 | 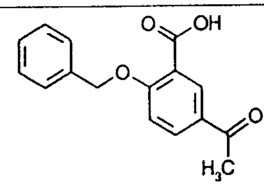 | C16H14O4 | 271 | 3 |

| | | | | |
|---|---|---|---|---|
| 281 | 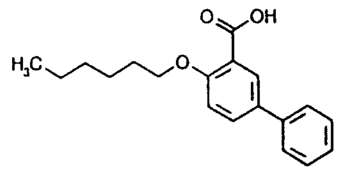 | C19H22O3 | 299 | 3 |
| 282 | 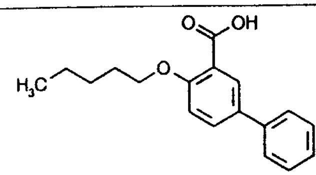 | C18H20O3 | 285 | 3 |
| 283 | 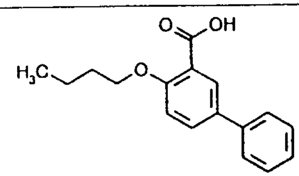 | C17H18O3 | 271 | 3 |
| 284 | 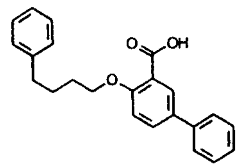 | C23H22O3 | 347 | 3 |
| 285 | 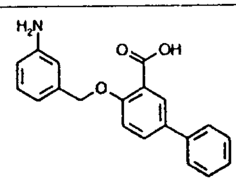 | C20H17NO3 | 320 | 3 |
| 286 | 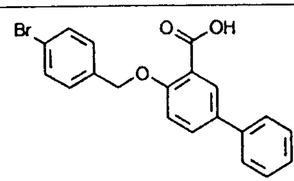 | C20H15BrO3 | 384 | 3 |

| | | | | |
|---|---|---|---|---|
| 287 | 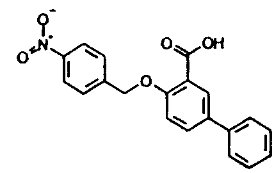 | C20H15NO5 | 350 | 3 |
| 288 | 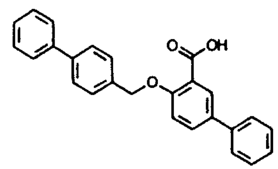 | C26H20O3 | 381 | 3 |
| 289 | 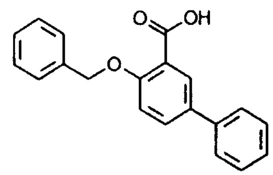 | C20H16O3 | 305 | 3 |
| 290 | 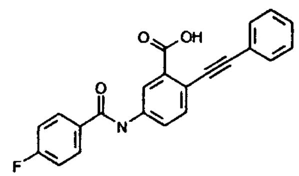 | C22H14FNO3 | 360 | 1 |
| 291 | 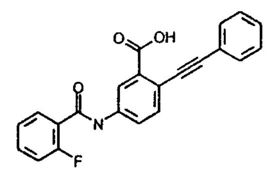 | C22H14FNO3 | 360 | 1 |
| 292 | 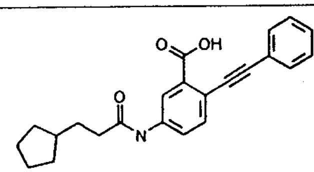 | C23H23NO3 | 362 | 1 |

| | | | | |
|---|---|---|---|---|
| 293 | 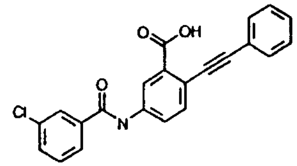 | C22H14ClNO3 | 376 | 1 |
| 294 | 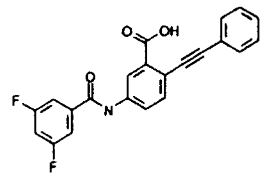 | C22H13F2NO3 | 378 | 1 |
| 295 | 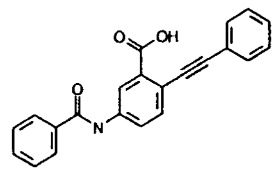 | C22H15NO3 | 342 | 1 |
| 296 | 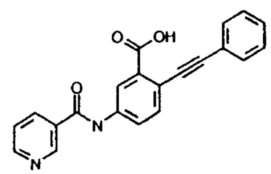 | C21H14N2O3 | 343 | 1 |
| 297 | 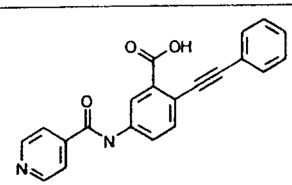 | C21H14N2O3 | 343 | 1 |
| 298 | 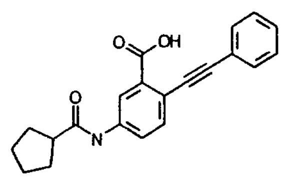 | C21H19NO3 | 334 | 1 |

| | | | | |
|---|---|---|---|---|
| 299 | 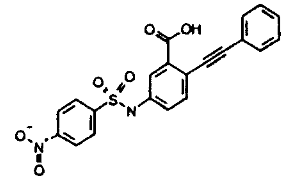 | C21H14N2O6S | 423 | 1 |
| 300 | 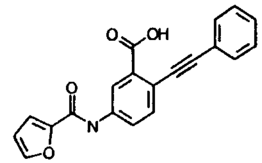 | C20H13NO4 | 332 | 1 |
| 301 | 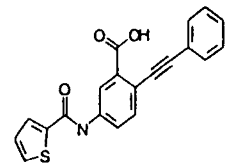 | C20H13NO3S | 348 | 1 |
| 302 | 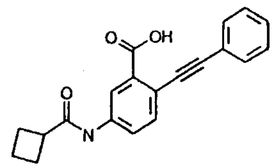 | C20H17NO3 | 320 | 1 |
| 303 | 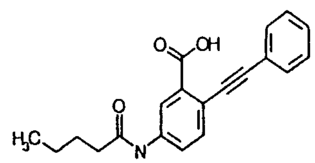 | C20H19NO3 | 322 | 1 |
| 304 | 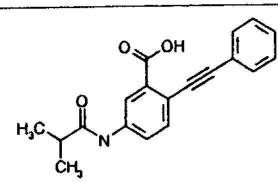 | C19H17NO3 | 308 | 1 |

| | | | | |
|---|---|---|---|---|
| 305 | 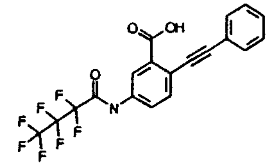 | C19H10F7NO3 | 434 | 1 |
| 306 | 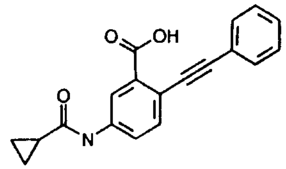 | C19H15NO3 | 306 | 1 |
| 307 | 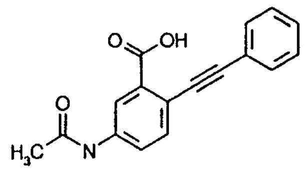 | C17H13NO3 | 280 | 1 |
| 308 | 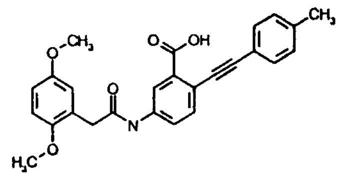 | C26H23NO5 | 430 | 1 |
| 309 | 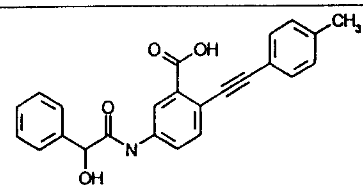 | C24H19NO4 | 386 | 1 |
| 310 | 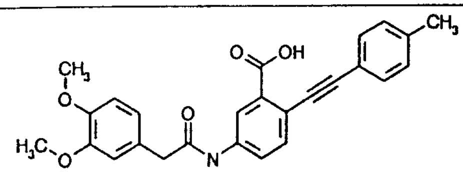 | C26H23NO5 | 430 | 1 |

| | | | | |
|---|---|---|---|---|
| 311 | 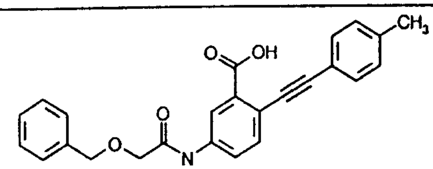 | C25H21NO4 | 400 | 1 |
| 312 | 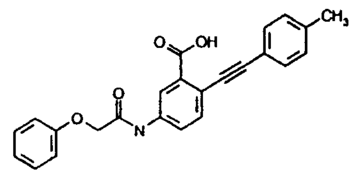 | C24H19NO4 | 386 | 1 |
| 313 | 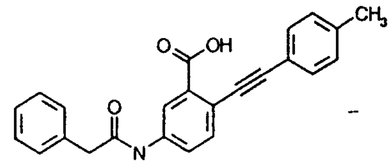 | C24H19NO3 | 370 | 1 |
| 314 | 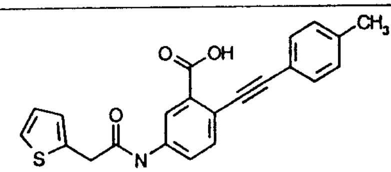 | C22H17NO3S | 376 | 1 |
| 315 | 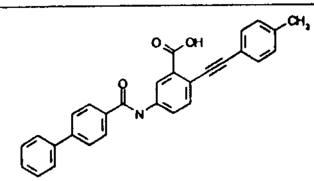 | C29H21NO3 | 432 | 1 |
| 316 | 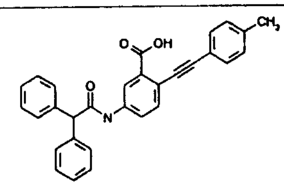 | C30H23NO3 | 446 | 1 |

| | | | | |
|---|---|---|---|---|
| 317 | 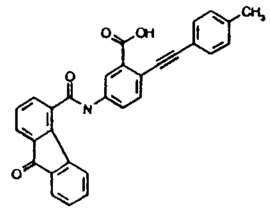 | C30H19NO4 | 458 | 1 |
| 318 | 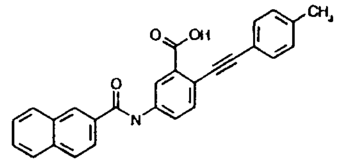 | C27H19NO3 | 406 | 1 |
| 319 | 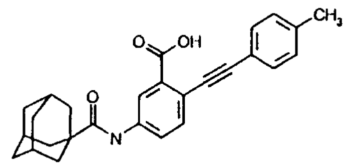 | C27H27NO3 | 414 | 1 |
| 320 | 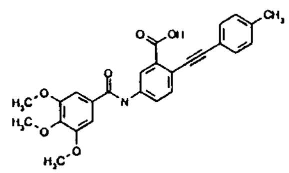 | C26H23NO6 | 446 | 1 |
| 321 | 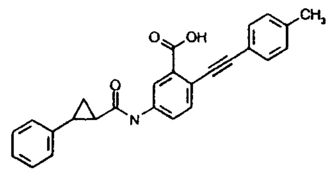 | C26H21NO3 | 396 | 1 |
| 322 | 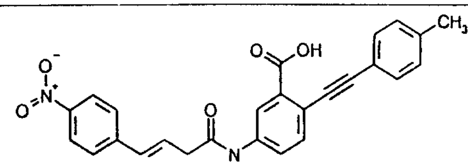 | C26H20N2O5 | 441 | 1 |

| | | | | |
|---|---|---|---|---|
| 323 | 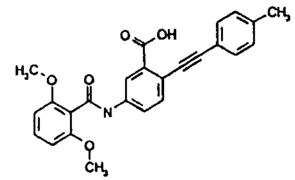 | C25H21NO5 | 416 | 1 |
| 324 | 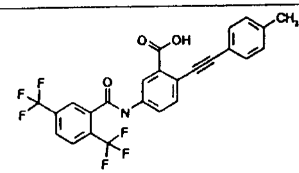 | C25H15F6NO3 | 492 | 1 |
| 325 | 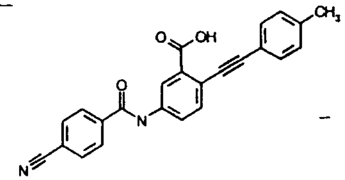 | C24H16N2O3 | 381 | 1 |
| 326 | 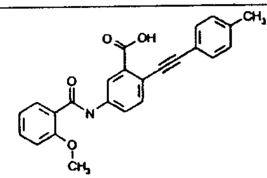 | C24H19NO4 | 386 | 1 |
| 327 | 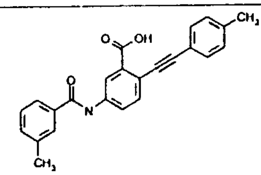 | C24H19NO3 | 370 | 1 |
| 328 | 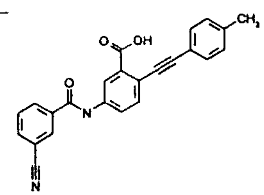 | C24H16N2O3 | 381 | 1 |

| | | | | |
|---|---|---|---|---|
| 329 | 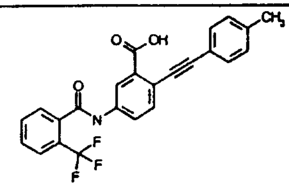 | C24H16F3NO3 | 424 | 1 |
| 330 | 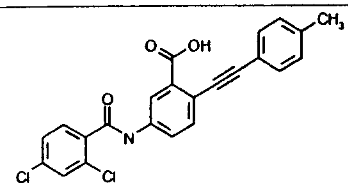 | C23H15Cl2NO3 | 425 | 1 |
| 331 | 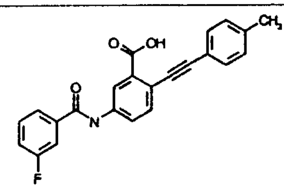 | C23H16FNO3 | 374 | 1 |
| 332 | 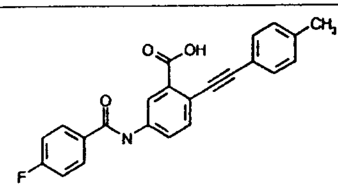 | C23H16FNO3 | 374 | 1 |
| 333 | 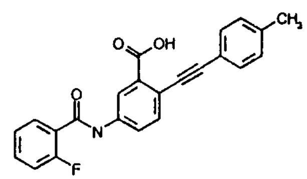 | C23H16FNO3 | 374 | 1 |
| 334 | 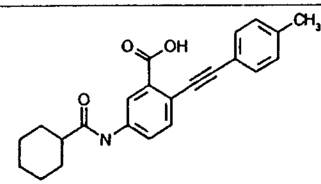 | C23H23NO3 | 362 | 1 |

| | | | | |
|---|---|---|---|---|
| 335 | 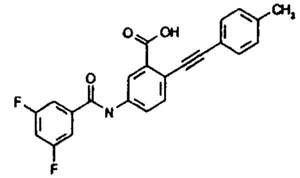 | C23H15F2NO3 | 392 | 1 |
| 336 | 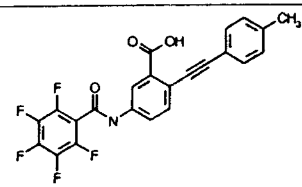 | C23H12F5NO3 | 446 | 1 |
| 337 | 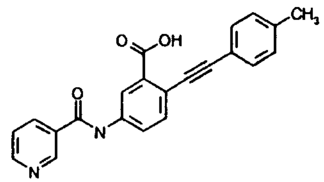 | C22H16N2O3 | 357 | 1 |
| 338 | 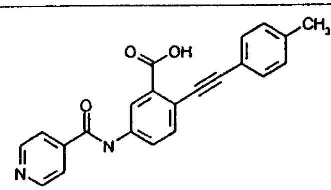 | C22H16N2O3 | 357 | 1 |
| 339 | 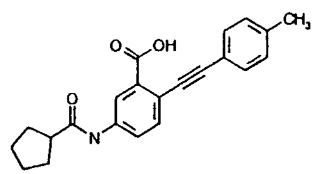 | C22H21NO3 | 348 | 1 |
| 340 | 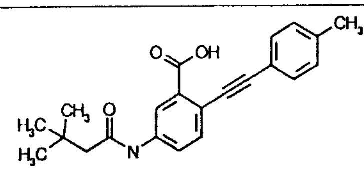 | C22H23NO3 | 350 | 1 |

| | | | | |
|---|---|---|---|---|
| 341 | 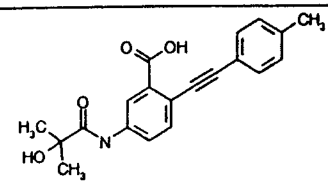 | C20H19NO4 | 338 | 1 |
| 342 | 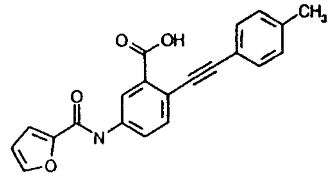 | C21H15NO4 | 346 | 1 |
| 343 | 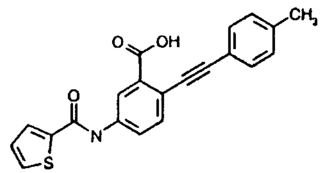 | C21H15NO3S | 362 | 1 |
| 344 | 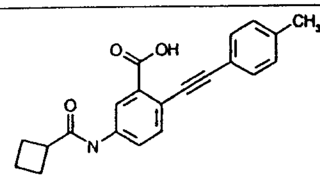 | C21H19NO3 | 334 | 1 |
| 345 | 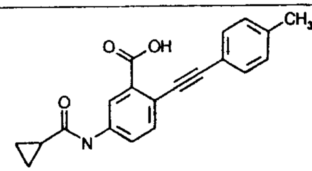 | C20H17NO3 | 320 | 1 |
| 346 | 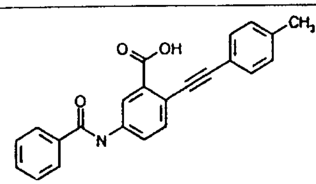 | C23H17NO3 | 356 | 1 |

| | | | | |
|---|---|---|---|---|
| 347 | 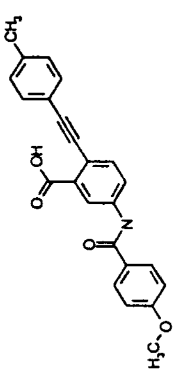 | C24H19NO4 | 386 | 1 |
| 348 | 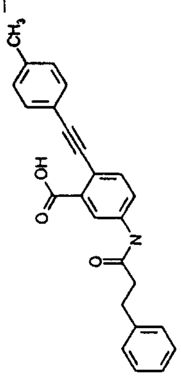 | C25H21NO3 | 384 | 1 |
| 349 | 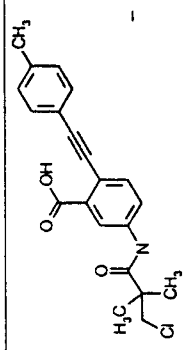 | C21H20ClNO3 | 370 | 1 |
| 350 | 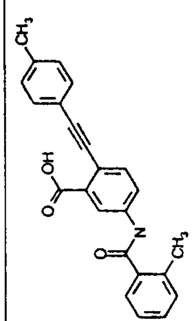 | C24H19NO3 | 370 | 1 |
| 351 | 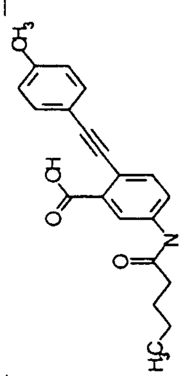 | C21H21NO3 | 336 | 1 |
| 352 | 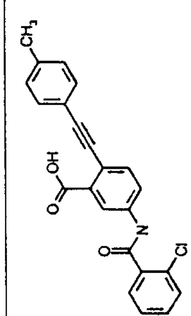 | C23H16ClNO3 | 390 | 1 |

**Patentansprüche**

**1.** Verbindungen der Formel I

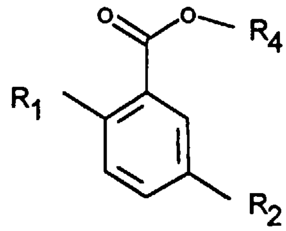

I

worin die Reste die folgende Bedeutung haben:

$R^1$

1. $C_1$-$C_{10}$-Alkyl,
2. $C_1$-$C_{10}$-Perfluoralkyl,
3. $C_1$-$C_{10}$-Hydroxyalkyl,
4. $C_2$-$C_{10}$-Alkenyl,
5. $C_2$-$C_{10}$-Alkinyl,
6. $C_1$-$C_{10}$-Alkyl-$C_6$-$C_{12}$-Aryl, wobei eine oder mehrere Methyleneinheiten durch O oder S ersetzt sein können,
7. $C_2$-$C_{10}$-Alkinyl-$C_6$-$C_{12}$-Aryl,
8. $C_6$-$C_{12}$-Aryl,
9. $C_1$-$C_9$-Heteroaryl,
10. $C_1$-$C_{10}$-Alkoxy, wobei der Alkylrest geradkettig oder verzweigt oder ab $C_3$-Alkoxy auch cyclisch sein kann,
11. O-$C_1$-$C_{10}$-Alkyl-$C_6$-$C_{12}$-Aryl, beispielsweise Benzyloxy,
12. einen Rest 1., 4., 5., 6., 7., 8., 9. substituiert mit einem oder mehreren Resten aus der Reihe CN, Nitro, Trifluormethyl, Halogen, Hydroxy-, Phenyl, $NH_2$, NH($C_1$-$C_{10}$-alkyl), N($C_1$-$C_{10}$-alkyl)$_2$, $C_1$-$C_{10}$-Alkoxy- (wobei der Alkylrest geradkettig oder verzweigt, oder ab $C_3$-Alkoxy auch cyclisch sein kann);

$R^2$

1. $C_1$-$C_{10}$-Alkyl,
2. $C_2$-$C_{10}$-Alkenyl,
3. $C_2$-$C_{10}$-Alkinyl,
4. $C_1$-$C_{10}$-Alkyl-$C_6$-$C_{12}$-Aryl,
5. $C_2$-$C_{10}$-Alkinyl-$C_6$-$C_{12}$-Aryl,
6. $C_6$-$C_{12}$-Aryl,
7. $C_2$-$C_{10}$-Alkinyl-$C_3$-$C_8$-Cycloalkenyl,
8. $C_1$-$C_9$-Heteroaryl;
9. NH-C0-$R^3$,
10. $NHSO_2$-$C_6$-$C_{12}$-Aryl, wobei der Aryl-Teil gegebenenfalls mit 1, 2 oder 3 Resten aus der Reihe $NO_2$, Halogen, Trifluormethyl, CN, NH($C_1$-$C_{10}$-Alkyl), N($C_1$-$C_{10}$-Alkyl)$_2$, $C_1$-$C_{10}$-Alkoxy, Hydroxy substituiert ist,
11. $C_1$-$C_{10}$-Alkanoyl;
12. einen Rest 1. - 8. ein- oder mehrfach substituiert mit $NH_2$, NH($C_1$-$C_{10}$-alkyl), N($C_1$-$C_{10}$-alkyl)$_2$, Carboxy, Halogen, Hydroxy, $C_1$-$C_6$-Alkoxy;

$R^3$

1. $C_1$-$C_{10}$-Alkyl,
2. $C_1$-$C_{10}$-Alkyl-$C_6$-$C_{12}$-Aryl,
3. $C_1$-$C_{10}$-Alkyl-($C_6$-$C_{12}$-Aryl)$_2$,
4. $C_1$-$C_{10}$-Alkyl-$C_1$-$C_9$-Heteroaryl,
5. $C_1$-$C_{10}$-Alkyl-($C_1$-$C_9$-Heteroaryl)$_2$,

6. NH-$C_6$-$C_{12}$-Aryl,
7. NH-$C_1$-$C_9$-Heteroaryl,
9. $C_2$-$C_{10}$-Alkenyl-$C_6$-$C_{12}$-Aryl,
10. $C_6$-$C_{12}$-Aryl,
11. $C_3$-$C_8$-Cycloalkyl,
12. $C_3$-$C_8$-Cycloalkyl-$C_6$-$C_{12}$-aryl,
13. $C_1$-$C_9$-Heteroaryl,
14. $C_1$-$C_{10}$-Alkyl-$C_3$-$C_8$-cycloalkyl,
15. Adamantyl,
16. C-O-$C_1$-$C_{10}$-Alkyl-$C_6$-$C_{12}$-Aryl, z.B. C-O-Benzyl,
17. C-O-$C_6$-$C_{12}$-Aryl, beispielsweise C-O-Phenyl,
18. Fluorenonyl

wobei die $C_6$-$C_{12}$-Aryl- und $C_1$-$C_9$-Heteroarylreste ein- oder mehrfach mit einem Rest aus der Reihe Hydroxy-, Halogen, $C_1$-$C_4$-Alkoxy, $NH_2$, NH($C_1$-$C_{10}$-alkyl), N($C_1$-$C_{10}$-alkyl)$_2$ $C_1$-$C_{10}$-Chloralkyl, $NO_2$, Carboxy, Carbamido, CN substituiert sein können;

$R^4$

1. Wasserstoff,
2. $C_1$-$C_{10}$-Alkyl

sowie deren physiologisch verträglichen Salze,
ausgenommen die Verbindungen der Formel I in denen gleichzeitig bedeuten:

a)

$R^1$ Phenyl,
$R^2$ NH-Acetyl
$R^4$ Wasserstoff

b)

$R^1$ Phenyl, disubstiuiert mit Nitro und Carboxy
$R^2$ NH-Acetyl
$R^4$ Wasserstoff

c)

$R^1$ Phenyl,
$R^2$ NH-Benzoyl
$R^4$ Wasserstoff

**2.** Verwendung der Verbindungen der Formel I

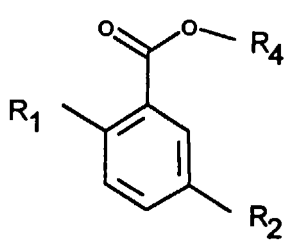

I

worin die Reste die folgende Bedeutung haben als Heilmittel.:

$R^1$

1. $C_1$-$C_{10}$-Alkyl,
2. $C_1$-$C_{10}$-Perfluoralkyl,
3. $C_1$-$C_{10}$-Hydroxyalkyl,
4. $C_2$-$C_{10}$-Alkenyl,
5. $C_2$-$C_{10}$-Alkinyl,
6. $C_1$-$C_{10}$-Alkyl-$C_6$-$C_{12}$-Aryl, wobei eine oder mehrere Methyleneinheiten durch O oder S ersetzt sein können,
7. $C_2$-$C_{10}$-Alkinyl-$C_6$-$C_{12}$-Aryl,
8. $C_6$-$C_{12}$-Aryl,
9. $C_1$-$C_9$-Heteroaryl,
10. $C_1$-$C_{10}$-Alkoxy, wobei der Alkylrest geradkettig oder verzweigt oder ab $C_3$-Alkoxy auch cyclisch sein kann,
11. O-$C_1$-$C_{10}$-Alkyl-$C_6$-$C_{12}$-Aryl, beispielsweise Benzyloxy,
12. einen Rest 1., 4., 5., 6., 7., 8., 9. substituiert mit einem oder mehreren Resten aus der Reihe CN, Nitro, Trifluormethyl, Halogen, Hydroxy-, Phenyl, $NH_2$, NH($C_1$-$C_{10}$-alkyl), N($C_1$-$C_{10}$-alkyl)$_2$, $C_1$-$C_{10}$-Alkoxy- (wobei der Alkylrest geradkettig oder verzweigt, oder ab $C_3$-Alkoxy auch cyclisch sein kann);

$R^2$

1. $C_1$-$C_{10}$-Alkyl,
2. $C_2$-$C_{10}$-Alkenyl,
3. $C_2$-$C_{10}$-Alkinyl,
4. $C_1$-$C_{10}$-Alkyl-$C_6$-$C_{12}$-Aryl,
5. $C_2$-$C_{10}$-Alkinyl-$C_6$-$C_{12}$-Aryl,
6. $C_6$-$C_{12}$-Aryl,
7. $C_2$-$C_{10}$-Alkinyl-$C_3$-$C_8$-Cycloalkenyl,
8. $C_1$-$C_9$-Heteroaryl;
9. NH-C0-$R^3$,
10. $NHSO_2$-$C_6$-$C_{12}$-Aryl, wobei der Aryl-Teil gegebenenfalls mit 1, 2 oder 3 Resten aus der Reihe $NO_2$, Halogen, Trifluormethyl, CN, NH($C_1$-$C_{10}$-Alkyl), N($C_1$-$C_{10}$-Alkyl)$_2$, $C_1$-$C_{10}$-Alkoxy, Hydroxy substituiert ist,
11. $C_1$-$C_{10}$-Alkanoyl;
12. einen Rest 1. - 8. ein- oder mehrfach substituiert mit $NH_2$, NH($C_1$-$C_{10}$-alkyl), N($C_1$-$C_{10}$-alkyl)$_2$, Carboxy, Halogen, Hydroxy, $C_1$-$C_6$-Alkoxy;

$R^3$

1. $C_1$-$C_{10}$-Alkyl,
2. $C_1$-$C_{10}$-Alkyl-$C_6$-$C_{12}$-Aryl,
3. $C_1$-$C_{10}$-Alkyl-($C_6$-$C_{12}$-Aryl)$_2$,
4. $C_1$-$C_{10}$-Alkyl-$C_1$-$C_9$-Heteroaryl,
5. $C_1$-$C_{10}$-Alkyl-($C_1$-$C_9$-Heteroaryl)$_2$,
6. NH-$C_6$-$C_{12}$-Aryl,
7. NH-$C_1$-$C_9$-Heteroaryl,
9. $C_2$-$C_{10}$-Alkenyl-$C_6$-$C_{12}$-Aryl,
10. $C_6$-$C_{12}$-Aryl,
11. $C_3$-$C_8$-Cycloalkyl,
12. $C_3$-$C_8$-Cycloalkyl-$C_6$-$C_{12}$-aryl,
13. $C_1$-$C_9$-Heteroaryl,
14. $C_1$-$C_{10}$-Alkyl-$C_3$-$C_8$-cycloalkyl,
15. Adamantyl,
16. C-O-$C_1$-$C_{10}$-Alkyl-$C_6$-$C_{12}$-Aryl, z.B. C-O-Benzyl,
17. C-O-$C_6$-$C_{12}$-Aryl, beispielsweise C-O-Phenyl,
18. Fluorenonyl

wobei die $C_6$-$C_{12}$-Aryl- und $C_1$-$C_9$-Heteroarylreste ein- oder mehrfach mit einem Rest aus der Reihe Hydroxy-,

Halogen, $C_1$-$C_4$-Alkoxy, $NH_2$, NH($C_1$-$C_{10}$-alkyl), N($C_1$-$C_{10}$-alkyl)$_2$ $C_1$-$C_{10}$-Chloralkyl, $NO_2$, Carboxy, Carbamido, CN substituiert sein können;

$R^4$

1. Wasserstoff,
2. $C_1$-$C_{10}$-Alkyl

sowie deren physiologisch verträglichen Salze.

**3.** Verwendung der Verbindungen der Formel I gemäß Anspruch 2 zur Behandlung von Krankheiten, die mit einer erhöhten Aktivität des Glucose-6-Phosphatase-Systems verbunden sind.

**4.** Verwendung der Verbindungen der Formel I gemäß Anspruch 2 zur Behandlung von Krankheiten, die mit einer erhöhten Glucoseproduktion der Leber verbunden sind.

**5.** Verwendung der Verbindungen der Formel I gemäß Anspruch 2 zur Behandlung des Typ II Diabetes (nicht insulinabhängiger oder Altersdiabetes).

**6.** Verwendung von Verbindungen nach Anspruch 1 oder 2 zur Herstellung von Arzneimitteln zur Behandlung des Typ II Diabetes und anderer Erkrankungen, die durch einen erhöhten Glucose-Ausstoß aus der Leber oder eine erhöhte Aktivität des Glucose-6-Phosphatase-Systems gekennzeichnet sind.

**7.** Arzneimittel enthaltend eine Verbindung der Formel I

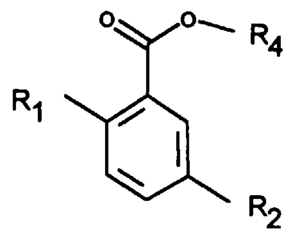

I

worin die Reste die folgende Bedeutung haben:

$R^1$

1. $C_1$-$C_{10}$-Alkyl,
2. $C_1$-$C_{10}$-Perfluoralkyl,
3. $C_1$-$C_{10}$-Hydroxyalkyl,
4. $C_2$-$C_{10}$-Alkenyl,
5. $C_2$-$C_{10}$-Alkinyl,
6. $C_1$-$C_{10}$-Alkyl-$C_6$-$C_{12}$-Aryl, wobei eine oder mehrere Methyleneinheiten durch O oder S ersetzt sein können,
7. $C_2$-$C_{10}$-Alkinyl-$C_6$-$C_{12}$-Aryl,
8. $C_6$-$C_{12}$-Aryl,
9. $C_1$-$C_9$-Heteroaryl,
10. $C_1$-$C_{10}$-Alkoxy, wobei der Alkylrest geradkettig oder verzweigt oder ab $C_3$-Alkoxy auch cyclisch sein kann,
11. O-$C_1$-$C_{10}$-Alkyl-$C_6$-$C_{12}$-Aryl, beispielsweise Benzyloxy,
12. einen Rest 1., 4., 5., 6., 7., 8., 9. substituiert mit einem oder mehreren Resten aus der Reihe CN, Nitro, Trifluormethyl, Halogen, Hydroxy-, Phenyl, $NH_2$, NH($C_1$-$C_{10}$-alkyl), N($C_1$-$C_{10}$-alkyl)$_2$, $C_1$-$C_{10}$-Alkoxy- (wobei der Alkylrest geradkettig oder verzweigt, oder ab $C_3$-Alkoxy auch cyclisch sein kann);

$R^2$

1. $C_1$-$C_{10}$-Alkyl,
2. $C_2$-$C_{10}$-Alkenyl,
3. $C_2$-$C_{10}$-Alkinyl,
4. $C_1$-$C_{10}$-Alkyl-$C_6$-$C_{12}$-Aryl,
5. $C_2$-$C_{10}$-Alkinyl-$C_6$-$C_{12}$-Aryl,
6. $C_6$-$C_{12}$-Aryl,
7. $C_2$-$C_{10}$-Alkinyl-$C_3$-$C_8$-Cycloalkenyl,
8. $C_1$-$C_9$-Heteroaryl;
9. NH-C0-$R^3$,
10. $NHSO_2$-$C_6$-$C_{12}$-Aryl, wobei der Aryl-Teil gegebenenfalls mit 1, 2 oder 3 Resten aus der Reihe $NO_2$, Halogen, Trifluormethyl, CN, NH($C_1$-$C_{10}$-Alkyl), N($C_1$-$C_{10}$-Alkyl)$_2$, $C_1$-$C_{10}$-Alkoxy, Hydroxy substituiert ist,
11. $C_1$-$C_{10}$-Alkanoyl;
12. einen Rest 1. - 8. ein- oder mehrfach substituiert mit $NH_2$, NH($C_1$-$C_{10}$-alkyl), N($C_1$-$C_{10}$-alkyl)$_2$, Carboxy, Halogen, Hydroxy, $C_1$-$C_6$-Alkoxy;

$R^3$

1. $C_1$-$C_{10}$-Alkyl,
2. $C_1$-$C_{10}$-Alkyl-$C_6$-$C_{12}$-Aryl,
3. $C_1$-$C_{10}$-Alkyl-($C_6$-$C_{12}$-Aryl)$_2$,
4. $C_1$-$C_{10}$-Alkyl-$C_1$-$C_9$-Heteroaryl,
5. $C_1$-$C_{10}$-Alkyl-($C_1$-$C_9$-Heteroaryl)$_2$,
6. NH-$C_6$-$C_{12}$-Aryl,
7. NH-$C_1$-$C_9$-Heteroaryl,
9. $C_2$-$C_{10}$-Alkenyl-$C_6$-$C_{12}$-Aryl,
10. $C_6$-$C_{12}$-Aryl,
11. $C_3$-$C_8$-Cycloalkyl,
12. $C_3$-$C_8$-Cycloalkyl-$C_6$-$C_{12}$-aryl,
13. $C_1$-$C_9$-Heteroaryl,
14. $C_1$-$C_{10}$-Alkyl-$C_3$-$C_8$-cycloalkyl,
15. Adamantyl,
16. C-O-$C_1$-$C_{10}$-Alkyl-$C_6$-$C_{12}$-Aryl, z.B. C-O-Benzyl,
17. C-O-$C_6$-$C_{12}$-Aryl, beispielsweise C-O-Phenyl,
18. Fluorenonyl

wobei die $C_6$-$C_{12}$-Aryl- und $C_1$-$C_9$-Heteroarylreste ein- oder mehrfach mit einem Rest aus der Reihe Hydroxy-, Halogen, $C_1$-$C_4$-Alkoxy, $NH_2$, NH($C_1$-$C_{10}$-alkyl), N($C_1$-$C_{10}$-alkyl)$_2$ $C_1$-$C_{10}$-Chloralkyl, $NO_2$, Carboxy, Carbamido, CN substituiert sein können;

$R^4$

1. Wasserstoff,
2. $C_1$-$C_{10}$-Alkyl

sowie deren physiologisch verträglichen Salze.

Europäisches Patentamt

**EUROPÄISCHER TEILRECHERCHENBERICHT**

der nach Regel 45 des Europäischen Patentübereinkommens für das weitere Verfahren als europäischer Recherchenbericht gilt

Nummer der Anmeldung
EP 97 10 8869

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | K. CHEBAANE ET AL.: "Synthèse d'aryl-2 naphthalènes et de dibenzocoumarines. 2o partie"<br>BULLETIN DE LA SOCIETE CHIMIQUE DE FRANCE.,<br>1975, PARIS FR,<br>Seiten 2521-2526, XP002041850<br>* Seite 2525; Beispiel 13D * | 1 | C07C233/55<br>C07C235/38<br>A61K31/165<br>A61K31/19<br>C07D333/40<br>C07C275/42<br>C07C233/63<br>C07D307/68<br>C07D333/28<br>C07C233/81<br>C07C233/54<br>C07C235/24<br>C07C235/56<br>C07C255/57 |
| X | US 3 948 937 A (JOHNSON ALEXANDER LAWRENCE ET AL) 6.April 1976<br>* Spalte 15 - Spalte 16; Beispiel 23 * | 1 | |
| X | L. BOUYAZZA ET AL.: "Synthèse de pyrrolo[1,2-a]quinoleines"<br>JOURNAL OF HETEROCYCLIC CHEMISTRY.,<br>Bd. 28, Nr. 1, 1991, PROVO US,<br>Seiten 77-80, XP002041851<br>* Seite 77; Beispiel 7; Tabelle 1 * | 1 | |
| | -/-- | | RECHERCHIERTE SACHGEBIETE (Int.Cl.6)<br>C07C<br>C07D |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 29.September 1997 | Pauwels, G |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

**EUROPÄISCHER TEILRECHERCHENBERICHT**

Nummer der Anmeldung
EP 97 10 8869

| Kategorie | EINSCHLÄGIGE DOKUMENTE: Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | Y. TOBE ET AL.: "Unusual reactivity of [6]paracyclophane toward alkyllithiums" JOURNAL OF ORGANIC CHEMISTRY., Bd. 58, 1993, EASTON US, Seiten 5883-5885, XP002041852 * Seite 5884, linke Spalte; Beispiele 8C,8D * --- | 1 | |
| X | CHEMICAL ABSTRACTS, vol. 50, no. 5, 10.März 1956 Columbus, Ohio, US; Spalte 3317b; XP002041858 * Zusammenfassung; Beispiel IV * & M. YASHUE: "Reaction of substituted benzenes and chloral. II. Reaction of N-acetyl-p-toluidine and chloral" J.PHARM.SOC. JAPAN, Bd. 75, 1955, Seiten 615-20, --- | 1 | RECHERCHIERTE SACHGEBIETE (Int.Cl.6) |
| X | CHEMICAL ABSTRACTS, vol. 72, no. 19, 11.Mai 1970 Columbus, Ohio, US; abstract no. 100967, XP002041859 * Zusammenfassung * & LYUSHIN ET AL. : "Synthesis of some substituted benzoic acids" NEFTEKHIMIYA, Bd. 9, Nr. 6, 1969, Seiten 900-904, * Seite 903; Beispiel 5; Tabelle 4 * --- | 1 | |
| | -/-- | | |

EPO FORM 1503 03.82 (P04C12)

Europäisches Patentamt

# EUROPÄISCHER TEILRECHERCHENBERICHT

Nummer der Anmeldung
EP 97 10 8869

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | A. NEWTON: "Polyisopropylbenzenes. IV. Bromo derivatives, nitriles, amides and carboxylic acids" JOURNAL OF THE AMERICAN CHEMICAL SOCIETY., Bd. 65, 1943, DC US, Seiten 2441-2443, XP002041853 * Seite 2443; Tabellen III,IV * --- | 1 | |
| X | CH.T. LESTER ET AL.: "Studies in p-cymene. I. The saponification rate of isomeric benzoates derived from p-cymene" JOURNAL OF THE AMERICAN CHEMICAL SOCIETY., Bd. 68, 1946, DC US, Seiten 375-376, XP002041854 * Seite 376; Tabelle I * --- | 1 | RECHERCHIERTE SACHGEBIETE (Int.Cl.6) |
| X | E.P. TAYLOR ET AL.: "The synthesis of some 5-alkyl-2-methylbenzoic acids" JOURNAL OF THE CHEMICAL SOCIETY, 1952, LETCHWORTH GB, Seiten 1123-1126, XP002041855 * Seite 1123; Beispiele I-V,VIII-X * --- | 1 | |
| X | D. PELTIER ET AL.: "Étude de la structure des acétanilides substitués par spectrographie infrarouge, (2e mémoire) Les esters benzoïques acétaminés" BULLETIN DE LA SOCIETE CHIMIQUE DE FRANCE., 1961, PARIS FR, Seiten 1621-1623, XP002041856 * Seite 1622; Tabelle I * --- | 1 | |
| | -/-- | | |

EPO FORM 1503 03.82 (P04C12)

Europäisches Patentamt

**EUROPÄISCHER TEILRECHERCHENBERICHT**

Nummer der Anmeldung
EP 97 10 8869

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | F.G. BADDAR ET AL.: "3:4-Benzfluorenones. Part II. Further observations on the Effect of groups on their fission with alkali" JOURNAL OF THE CHEMICAL SOCIETY., 1948, LETCHWORTH GB, Seiten 1231-1235, XP002041857 * Seite 1231; Beispiel III * --- | 1 | |
| X | O. JACOBSEN: "Über die dritee Xylylsäure un ihr entsprechende Xylidinsäure" BERICHTE DER DEUTSCHEN CHEMISCHEN GESELLSCHAFT., 1881, WEINHEIM DE, Seiten 2110-2116, XP002042072 * Seite 2111, Absatz 3 * --- | 1 | RECHERCHIERTE SACHGEBIETE (Int.Cl.6) |
| D,A | EP 0 587 088 A (HOECHST AG) 16.März 1994 ----- | | |

EPO FORM 1503 03.82 (P04C12)

Europäisches Patentamt

## UNVOLLSTÄNDIGE RECHERCHE
## ERGÄNZUNGSBLATT C

Nummer der Anmeldung
EP 97 10 8869

Vollständig recherchierte Ansprüche:
-

Unvollständig recherchierte Ansprüche:
1 - 7

Grund für die Beschränkung der Recherche:

The search revealed a large number of particular relevant compounds disclosed in a large number of individual publications, a complete search report is therefore not possible. A limited number of documents, illustrating the prior art has been cited in the search report.